# EUROPÄISCHE PATENTSCHRIFT

(11) **EP 3 384 866 B1**
(45) Veröffentlichungstag und Bekanntmachung des Hinweises auf die Patenterteilung: **08.01.2020**
(21) Anmeldenummer: 18165523.4
(22) Anmeldetag: 03.04.2018
(51) Int. Cl.: A61B 17/88, B01F 3/12, A61M 5/28, A61M 5/315, B01F 5/06, B01F 13/00, B01F 15/02

(54) **VORRICHTUNG ZUM LAGERN, VERMISCHEN UND AUSTRAGEN EINES KNOCHENZEMENTS UND VERFAHREN HIERZU**
DEVICE FOR STORAGE, MIXING AND DISPENSING OF A BONE CEMENT, AND PERTINENT METHOD
DISPOSITIF DE STOCKAGE, DE MÉLANGE ET DE DISTRIBUTION D'UN CIMENT OSSEUX ET PROCÉDÉ CORRESPONDANT

(30) Priorität: 07.04.2017 DE 102017107569
(43) Veröffentlichungstag der Anmeldung: 10.10.2018
(73) Patentinhaber: Heraeus Medical GmbH, 61273 Wehrheim (DE)
(72) Erfinder: Vogt, Sebastian, 99092 Erfurt (DE); Kluge, Thomas, 56179 Vallendar (DE)
(74) Vertreter: Schultheiss & Sterzel Patentanwälte PartG mbB

(56) Entgegenhaltungen:
- JP-A- 2011 067 265
- US-A- 3 682 174
- US-A- 4 613 326
- US-A- 4 994 065
- US-A1- 2003 014 056
- US-A1- 2011 027 751

## Beschreibung

Die Erfindung betrifft eine Vorrichtung zum Lagern einer Monomerflüssigkeit und eines Zementpulvers als Ausgangskomponenten eines Knochenzementteigs sowie zum Mischen des Knochenzementteigs aus den Ausgangskomponenten und zum Austragen des gemischten Knochenzementteigs.

Die Erfindung betrifft auch eine Auspressvorrichtung zum Anwenden einer solchen Vorrichtung und ein Verfahren zur Herstellung eines Knochenzementteigs, insbesondere eines pastenförmigen Polymethylmethacrylat-Knochenzementteigs.

Gegenstand der Erfindung ist insbesondere eine Vorrichtung zum separaten Lagern des Zementpulvers und der Monomerflüssigkeit von Polymethylmethacrylat-Knochenzement, zum anschließenden Vermischen des Zementpulvers mit der Monomerflüssigkeit zur Bildung eines Knochenzementteigs und zum Austragen des gemischten Knochenzementteigs. Der mit der Vorrichtung hergestellte Knochenzementteig ist insbesondere für die Augmentation von frakturierten Wirbelkörpern bestimmt, also für die Vertebroplastie oder Kyphoplastie. Es handelt sich bei der erfindungsgemäßen Vorrichtung um ein Full-Prepacked-Zementiersystem.

Polymethylmethacrylat-(PMMA)-Knochenzemente gehen auf die grundlegenden Arbeiten von Sir Charnley zurück (Charnley, J.: Anchorage of the femoral head prosthesis of the shaft of the femur. J. Bone Joint Surg. 42 (1960) 28-30.). Konventionelle Polymethylmethacrylat-Knochenzemente (PMMA-Knochenzemente) sind aus einer pulverförmigen Komponente und einer flüssigen Monomerkomponente zusammengesetzt (K.-D. Kühn: Knochenzemente für die Endoprothetik: Ein aktueller Vergleich der physikalischen und chemischen Eigenschaften handelsüblicher PMMA-Zemente. Springer-Verlag Berlin Heidelberg New York, 2001). Die Pulverkomponente, auch als Zementpulver oder Knochenzementpulver bezeichnet, weist ein oder mehrere Polymere auf, die auf Basis von Methylmethacrylat und Comonomeren, wie Styren, Methylacrylat oder ähnlichen Monomeren durch Polymerisation, vorzugsweise Suspensionspolymerisation, hergestellt sind, einen Röntgenopaker und den Initiator Dibenzoylperoxid auf. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylats. Mit fortschreitender Polymerisation des Methylmethacrylats erhöht sich die Viskosität des Zementteigs, bis dieser erstarrt.

Für die Behandlung von Impressionsfrakturen von Wirbelkörpern wurde eine Reihe von speziellen Polymethylmethacrylat-Knochenzementen entwickelt. Diese zeichnen sich dadurch aus, dass sie einen relativ hohen Anteil an Röntgenopakern, zum Beispiel Zirkoniumdioxid oder Bariumsulfat, enthalten. Dadurch soll eine fortlaufende Kontrolle der Ausbreitung des Knochenzementteigs im frakturierten Wirbel durch Fluoroskopie erleichtert werden. Die gegenwärtig hauptsächlich angewandten Verfahren zur Augmentation von frakturierten Wirbelkörpern sind die Vertebroplastie und die Kyphoplastie. Bisher ist dabei die manuelle Vermischung der Zementkomponenten in Mischbechern oder in einfachen Mischvorrichtungen üblich.

PMMA-Knochenzemente können in geeigneten Mischbechern mit Hilfe von Spateln durch Vermischen des Zementpulvers mit der Monomerflüssigkeit vermischt werden. Dabei kann es zum Einschluss von Luftblasen im Knochenzementteig kommen, welche die mechanischen Eigenschaften des ausgehärteten Knochenzements negativ beeinflussen können.

Zur Vermeidung von Lufteinschlüssen im Knochenzementteig wurden eine Vielzahl von Vakuum-Zementiersystemen beschrieben, von denen exemplarisch folgende genannt sind: US 6 033 105 A, US 5 624 184 A, US 4 671 263 A, US 4 973 168 A, US 5 100 241 A, WO 99/67015 A1, EP 1 020 167 A2, US 5 586 821 A, EP 1 016 452 A2, DE 36 40 279 A1, WO 94/26403 A1, EP 1 005 901 A2, EP 1 886 647 A1, US 5 344 232 A.

Eine Weiterentwicklung in der Zementiertechnik stellen Zementiersysteme dar, in denen sowohl das Zementpulver als auch die Monomerflüssigkeit bereits in separaten Kompartimenten der Mischvorrichtungen verpackt sind und die erst unmittelbar vor der Zementapplikation im Zementiersystem miteinander vermischt werden. Solche geschlossenen Full-Prepacked-Mischvorrichtungen wurden mit der EP 0 692 229 A1, der DE 10 2009 031 178 B3, der US 5 997 544 A, der US 6 709 149 B1, der DE 698 12 726 T2, der EP 0 796 653 A2 und der US 5 588 745 A vorgeschlagen.

Das Patent DE 10 2009 031 178 B3 offenbart eine Lager- und Mischvorrichtung als Full-Prepacked-Mischvorrichtung, in dem die zur Herstellung des Knochenzementteigs notwendigen Ausgangskomponenten bereits in der Lager- und Mischvorrichtung gelagert sind und in der Lager- und Mischvorrichtung zusammengeführt und gemischt werden können. Die Lager- und Mischvorrichtung weist einen zweiteiligen Austragskolben zum Verschluss einer Zementkartusche auf. Dabei wird eine Kombination aus einem gasdurchlässigen Sterilisationskolben und einem gasundurchlässigen Dichtungskolben verwendet.

Polymethylmethacrylat-Knochenzemente werden nach Vermischung des Zementpulvers mit der flüssigen Monomerkomponente im noch nicht ausgehärteten, pastenförmigen Zustand als Zementteig appliziert. Bei Verwendung von Mischvorrichtungen befindet sich der Zementteig im Fall von Pulver-Flüssigkeits-Zementen in einer Kartusche. Aus dieser Kartusche wird der Zementteig durch Bewegung eines Austragskolbens herausgedrückt. Die Austragskolben haben üblicherweise einen Durchmesser zwischen 30 mm bis 40 mm und damit an der Außenseite, an welcher der Stößel beziehungsweise eine Stange der Auspressvorrichtung beim Auspressvorgang angreift, eine Fläche von 7,0 cm² bis 12,5 cm². Die Bewegung des Austragskolbens wird durch manuell bedienbare, mechanische Auspressvorrichtungen bewirkt, die auch als Applikatoren bezeichnet werden. Diese manuellen Auspressvorrichtungen erreichen normalerweise eine Auspresskraft im Bereich von ungefähr 1,5 kN bis 3,5 kN.

Bei der Applikation konventioneller PMMA-Knochenzemente, die aus einer flüssigen Monomerkomponente und einer separat dazu gelagerten Zementpulverkomponente als Ausgangskomponenten bestehen, wird nach der Vermischung der beiden Ausgangskomponenten in Zementiersystemen der gebildete Zementteig mit Hilfe von manuell bedienbaren Auspressvorrichtungen ausgepresst. Diese einfachen mechanischen Auspressvorrichtungen nutzen insbesondere Klemmstangen zum Auspressen, die durch einen manuell zu betätigenden Kipphebel angetrieben werden. Die manuell angetriebenen Auspressvorrichtungen sind seit Jahrzehnten weltweit erprobt und stellen bisher den Stand der Technik dar. Vorteilhaft an diesen Auspressvorrichtungen ist, dass der medizinische Anwender über die aufzubringende Handkraft ein Gefühl für den Eindringwiderstand des Knochenzementteigs in die Knochenstrukturen (Spongiosa) hat.

Bei der Verwendung aller bisher bekannten Full-Prepacked-Mischvorrichtungen muss der medizinische Anwender mehrere Arbeitsschritte in einer vorbestimmten Reihenfolge an den Vorrichtungen nacheinander durchführen bis der gemischte Knochenzementteig vorliegt und appliziert werden kann. Eine Verwechslung der Arbeitsschritte kann zum Versagen der Mischvorrichtung führen und daher Störungen im OP-Ablauf verursachen. Kostenintensive Schulungen der medizinischen Anwender sind deshalb notwendig, um Anwenderfehler zu vermeiden.

In der WO 00/35506 A1 wird eine Vorrichtung vorgeschlagen, bei der Polymethylmethacrylat-Zementpulver in einer Kartusche gelagert wird, wobei das Zementpulver das gesamte Volumen der Kartusche ausfüllt und die Zwischenräume zwischen den Partikeln des Zementpulvers ein solches Volumen hat, das dem Volumen der Monomerflüssigkeit entspricht, das zur Herstellung von Knochenzementteig mit dem in der Kartusche gelagerten Zementpulver notwendig ist. Diese Vorrichtung ist so aufgebaut, dass durch Vakuumeinwirkung die Monomerflüssigkeit von oben in die Kartusche eingeleitet wird, wobei hierzu ein Vakuum an einem Vakuumanschluss an der Unterseite der Kartusche angelegt wird. Dadurch wird die Monomerflüssigkeit durch das Zementpulver gezogen, wobei die in den Zwischenräumen der Zementpulverpartikel befindliche Luft durch die Monomerflüssigkeit verdrängt wird. Auf eine mechanische Durchmischung des gebildeten Zementteigs mit einem Rührer wird dabei verzichtet.

Nachteilig an diesem System ist, dass Zementpulver, die schnell mit der Monomerflüssigkeit anquellen, mit dieser Vorrichtung nicht angemischt werden können, weil die schnell anquellenden Zementpulverpartikel nach einem Eindringen der Monomerflüssigkeit in das Zementpulver von ungefähr 1 bis 2 cm eine gelartige Barriere bilden und die Migration der Monomerflüssigkeit durch das gesamte Zementpulver behindern. Konventionelle Zementpulver zeigen zudem das Phänomen, dass auf Grund der unterschiedlichen Oberflächenenergien die Zementpulverpartikel durch Methylmethacrylat nur schlecht benetzt werden. Dadurch dringt das Methylmethacrylat nur relativ langsam in das Zementpulver ein. Weiterhin kann bei Vakuumeinwirkung nicht ausgeschlossen werden, dass nach vollständiger Durchdringung des Zementpulvers durch die Monomerflüssigkeit die Monomerflüssigkeit über den Vakuumanschluss abgesaugt wird. Dann steht nicht genügend Monomerflüssigkeit für die Aushärtung durch radikalische Polymerisation zur Verfügung beziehungsweise das Mischungsverhältnis wird ungewollt verändert und damit auch die Konsistenz des Knochenzementteigs. Weiterhin ist es problematisch, dass die zwischen den Zementpulverpartikeln eingeschlossen Luft durch die Monomerflüssigkeit von oben nach unten verdrängt werden soll, weil die gegenüber der Monomerflüssigkeit spezifisch leichtere Luft auf Grund der Schwerkraft das Bestreben hat, im Zementpulver nach oben zu wandern und nicht nach unten in Richtung Vakuumanschluss zu migrieren.

Bei hochviskosen pastenförmigen Knochenzementen unter Verwendung von Kartuschen, bei denen der Austragskolben an der äußeren Kolbenseite, an der ein Stößel beziehungsweise eine Stange der Auspressvorrichtungen angreift, eine gesamte Fläche im Bereich von 7,0 cm² bis 12,5 cm² hat, sind solche Vorrichtungen nicht oder nur mit einem sehr großen Kraftaufwand manuell zu bedienen. Dies gilt insbesondere dann, wenn der Fließwiderstand des auszupressenden Knochenzementteigs durch einen Schlauch oder einen Trokar als verlängerte Austragsöffnung und/oder durch einen statischen Mischer erhöht wird, wie dies bei Anwendungen im Bereich der Wirbelsäule, wo der Knochenzementteig über einen Schlauch und einen Trokar ausgetragen wird, üblich ist. Dieser große Kraftaufwand ist medizinischen Anwendern im OP nicht zuzumuten.

Aus dem Kleb- und Dichtstoffbereich sind auch elektrisch angetriebene Auspressvorrichtungen bekannt. Diese Vorrichtungen können sowohl mit Akkumulatoren und mit Batterien als auch mit Hilfe einer stationären Stromversorgung angetrieben werden. Diese Vorrichtungen können mit ihren zum Teil sehr großen Auspresskräften besonders zähe pastenförmige Massen auspressen. Nachteilig ist jedoch bei der Verwendung von Elektromotoren, dass diese Buntmetalle enthalten und kostenintensiv in der Beschaffung sind.

Im steril zu haltenden OP-Bereich müssen derartige Vorrichtungen aufwendig sterilisiert oder sogar ausgetauscht werden. Bei einer elektrischen Verkabelung kann die Bewegung des Anwenders im OP behindert werden.

Weiterhin wurden auch pneumatische Vorrichtungen vorgeschlagen. Diese Geräte erfordern einen stationären oder mobilen Druckluftanschluss (US 2 446 501 A, DE 20 2005 010 206 U1). Dazu sind Druckluftschläuche notwendig, welche die Bewegung des Anwenders behindern können.

Alternativ dazu ist auch die Verwendung von Druckgaspatronen zur Bereitstellung von Druckgas möglich. Dazu wurden Vorrichtungen vorgeschlagen, bei denen der Druckgaszufluss durch ein Ventil und zusätzlich durch ein zweites Ventil der Strom der viskosen Masse gesteuert werden (US 2004/0074927 A1, US 6 935 541 B1). Bei diesen Vorrichtungen sind die Gaspatronen in den Vorrichtungen integriert. Bei derartigen an Druckluft angeschlossenen oder Druckgaspatronen enthaltenden Systemen ist immer eine Druckgasquelle erforderlich, ohne die die Systeme nicht mehr anwendbar sind.

In der Vertebroplastie wird die Applikation von Knochenzement mit einem Röntgenverfahren in situ verfolgt. Bei Applikationsvorrichtungen für die Vertebroplastie wird üblicherweise ein Schlauch eingesetzt, über dessen Spitze der Knochenzement appliziert werden kann, damit der Anwender außerhalb des Bereichs der Röntgenstrahlung arbeiten kann. Hierzu können am Schlauch des Weiteren ein Trokar oder eine Kanüle angeordnet sein. Derartige Systeme sind beispielsweise aus US 7 112 205 B2, US 8 038 682 B2, US 8 308 731 B2, DE 10 2005 045 227 A1, EP 1 074 231 B1, EP 1 596 736 B1, US 9 005 209 B2 und WO 2008/097855 A2 bekannt.

Alternativ können auch andere Aufbauten verwendet werden, um den Anwender von der Röntgenstrahlung fernzuhalten, wie beispielsweise in den Dokumenten US 6 676 663 B2, US 7 008 433 B2, US 8 348 494 B2, EP1 464 292 B1, EP 1 614 403 B1, US 2008/319445 A9 und WO 2008/038322 A2 beschrieben.

Ein Knochenzement-Applikator für die Vertebroplastie zum Applizieren von Knochenzement mit einem Schlauch, einem Trokar und einem Mischer ist aus der US 2008/0086143 A1 bekannt. Der Knochenzement-Applikator umfasst zwei nebeneinander angeordnete Kartuschen, in denen die Ausgangskomponenten auch gelagert werden. Der Knochenzement-Applikator wird unmittelbar vor der Anwendung zusammengesetzt. Bei derartigen Knochenzement-Applikatoren für die Vertebroplastie wird auf die Ausgangskomponenten des Knochenzements mit Hilfe einer Auspressvorrichtung, die Austragskolben in den Kartuschen vortreiben, ein Druck ausgeübt und mit Hilfe des Drucks die Ausgangskomponenten aus den Kartuschen und durch den Schlauch ausgetrieben. Dabei werden die Ausgangskomponenten meist zuvor in einem vorgeschalteten statischen Mischer gemischt. Dies führt dazu, dass sich die den Knochenzementfluss begrenzenden Teile des Knochenzement-Applikators (die Kartuschen, das Gehäuse des Mischers und der Schlauch) elastisch verformen. Wenn der Vortrieb der Austragskolben gestoppt wird, kommt es durch die elastische Kraft dieser Teile zu einer Volumenkontraktion dieser Teile, so dass auch dann noch Knochenzement durch die Applikationsöffnung des Schlauchs beziehungsweise Trokars austritt. Hierdurch kann es zu einer Kontamination des OP-Saals oder des Anwenders mit dem Knochenzement kommen oder es wird ungewollt eine zu große Menge des Knochenzements appliziert. Zudem muss, wenn der Volumenstrom des Knochenzementteigs wieder gestartet werden soll, zunächst wieder der Druck im Knochenzement aufgebaut werden, damit dieser durch die Applikationsöffnung austritt. Dadurch verzögert sich wiederum der Zeitpunkt nach dem Beginn des Vortreibens der Austragskolben, ab dem tatsächlich der Knochenzementteig appliziert werden kann, was ebenfalls unerwünscht ist. Aufgrund der großen Zähigkeit des Knochenzementteigs und der Ausgangskomponenten, insbesondere, wenn pastöse Ausgangskomponenten verwendet werden, sind alle diese Effekte relativ stark ausgeprägt. Dem kann durch die Verwendung massiver und teurer metallisches Gehäuseteile entgegengewirkt werden. Diese Teile müssen nach der Anwendung gereinigt und für eine weitere Anwendung sterilisiert werden oder sie müssen aufwendig recycelt werden. Zudem können beim Lösen der Kartuschen Reste der Ausgangskomponenten freigesetzt werden und dadurch den OP-Saal kontaminieren.

Die US 8,544,683 B2 offenbart ein Kartuschensystem, das zur Beimischen einer geringen Menge zu einer Hauptausgangskomponente geeignet ist. Bei dem Kartuschensystem ist neben einer Kartusche eine zweite kleinere Kartusche angeordnet, wobei bei einem Vortrieb eines Austragskolbens in der größeren Kartusche auch ein Austragskolben in der kleineren Kartusche über ein gemeinsames Verbindungselement angetrieben wird. Das System ist zum Mischen der zähen pastösen Ausgangskomponenten von PMMA-Knochenzement jedoch nicht geeignet.

Weitere Vorrichtungen der oben genannten Art sind aus der US 2003/014056 und der US 4994065 bekannt.

Die Aufgabe der vorliegenden Erfindung besteht also darin, die Nachteile des Stands der Technik zu überwinden. Insbesondere besteht die Aufgabe der Erfindung in der Entwicklung einer Vorrichtung, die zum Lagern einer Monomerflüssigkeit und eines Zementpulvers als Ausgangskomponenten eines Knochenzementteigs sowie zum Mischen des Knochenzementteigs aus den Ausgangskomponenten und zum Austragen des gemischten Knochenzementteigsund geeignet ist, einer Auspressvorrichtung, die zur Anwendung der Vorrichtung geeignet ist sowie eines Verfahrens zur Herstellung eines Knochenzementteigs, insbesondere eines pastenförmigen Polymethylmethacrylat-Knochenzementteigs, wobei der Knochenzementteig aus einem Zementpulver und einer Monomerflüssigkeit mit einer solchen Vorrichtung hergestellt wird.

Die Vorrichtung soll durch eine einfache Auspressvorrichtung anzutreiben sein und dabei möglichst einfach in der Bedienung sein. Der Aufbau soll kostengünstig sein, damit die Vorrichtung aus hygienischen Gründen nur einmalig verwendet werden kann. Es sollen möglichst viele oder alle in der Vorrichtung ablaufenden Prozesse, wie die Vermischung der Ausgangskomponenten, das Austragen des Knochenzementteigs und das Öffnen von Behältern und gegebenenfalls der Kartusche, mit möglichst wenigen Arbeitsschritten und so weit als möglich automatisiert ablaufen und vorzugsweise mit einem einzigen Antrieb anzutreiben sein.

Die Aufgabe der Erfindung besteht also auch in der Entwicklung einer Vorrichtung zum Lagern, Vermischen von Zementpulver und Monomerflüssigkeit, wobei der durch Vermischung der Zementkomponenten gebildete Polymethylmethacrylat-Knochenzement zur Augmentation von frakturierten Wirbelkörpern bestimmt ist. Die Handhabung der Vorrichtung soll maximal vereinfacht sein, um Anwendungsfehler infolge von fehlerhaft durchgeführten Montageschritten grundsätzlich zu vermeiden. Der medizinische Anwender soll die Vorrichtung nach der Entnahme aus einer Verpackung mit einer Auspressvorrichtung verbinden und diese anschließend betätigen. Weitere Montage- und Arbeitsschritte sollen durch den Aufbau der Vorrichtung vermieden werden. Die Vorrichtung soll eine sichere Lagerung von Zementpulver und Monomerflüssigkeit in voneinander getrennten Kompartimenten ermöglichen, so dass während der Lagerung der Vorrichtung eine unbeabsichtigte Vermischung der Zementkomponenten ausgeschlossen ist. Die Vorrichtung soll eine Sterilisation mit dem Gas Ethylenoxid ermöglichen. Das in der Vorrichtung gelagerte Zementpulver muss dazu für Ethylenoxid zugänglich sein. Die Vorrichtung soll mit Hilfe einer im OP manuell angetriebenen Auspressvorrichtung aktivierbar sein, so dass nach der form- oder kraftschlüssigen Verbindung der Vorrichtung mit der Auspressvorrichtung durch Betätigung der Auspressvorrichtung die axial vortreibbare Stange der Auspressvorrichtung auf die Vorrichtung einwirkt und den Monomerflüssigkeitsbehälter öffnet und anschließend bei weiterer Bewegung der Stange die Monomerflüssigkeit in das Zementpulver transferiert. Die Vermischung der Monomerflüssigkeit mit dem Zementpulver soll ohne einen von außen manuell zu bewegenden Mischer erfolgen. Es soll möglichst nur mit der Vorwärtsbewegung der Stange der Auspressvorrichtung das Öffnen des Monomerflüssigkeitsbehälters, der nachfolgende Monomerflüssigkeitstransfer in das Zementpulver und die Vermischung der Zementkomponenten unter Bildung des Knochenzementteigs vorgenommen werden. Weiterhin ist es für eine Anwendung in der Vertebroplastie wichtig, dass an der Vorrichtung ein geeigneter Konnektor oder ein Konnektor mit einem Schlauch angebracht werden kann, durch die der gebildete Knochenzementteig appliziert werden kann. Es ist für solche Anwendungen weiterhin wichtig, dass der Kolben zum Austrag des gebildeten Knochenzementteigs so gestaltet ist, dass bei der Verwendung einer manuellen Auspressvorrichtung der Druck auf den Kolben so hoch ist, dass der Knochenzementteig durch einen Kunststoffschlauch mit einem Innendurchmesser von 3 mm mindestens über eine Strecke von 20 cm ausgepresst werden kann.

Bevorzugt soll auch eine einfache und kostengünstig zu fertigende Vorrichtung als Knochenzement-Applikator für die Vertebroplastie für pastenförmige Mehrkomponenten-Polymethylmethacrylat-Knochenzemente und ein Verfahren zum Applizieren eines Knochenzementteigs mit einer solchen Vorrichtung bereitgestellt werden, die einfach aufgebaut ist und kostengünstig zu fertigen ist, wobei es beim Stoppen oder Unterbrechen des Flusses des Knochenzementteigs nicht zu einem Nachlaufen des Knochenzementteigs kommen soll. Ferner soll die Vorrichtung nach Unterbrechung des Flusses des Knochenzementteigs möglichst schnell wieder einsetzbar sein. Es soll eine Kontamination der Umgebung und des Anwenders mit Knochenzementteig oder den Ausgangskomponenten, insbesondere mit der Monomerflüssigkeit möglichst ausgeschlossen werden.

Die Vorrichtung soll möglichst einfach aus Kunststoff gefertigt werden können und sich dadurch als Produkt zur einmaligen Anwendung eignen. Das Auspressen des gemischten Zementteigs soll mit einer konventionellen, manuell angetriebenen Auspressvorrichtung, wie sie bisher bei PMMA-Knochenzementen zur Zementierung von Knie- und Hüft-TEP (totale Endoprothese des Hüftgelenks) üblich ist, möglich sein. Der Knochenzement-Applikator soll so beschaffen sein, dass ein sofortiger Notstop des fließenden Knochenzementteigs möglich ist, ohne dass eine Kontamination des OPs (Operationssaals) mit dem Knochenzementteig beziehungsweise ein Weiterfließen des Knochenzementteigs erfolgt.

Bevorzugt soll der zu entwickelnde Knochenzement-Applikator keine zwei miteinander verbundenen und synchron vorzutreibenden Stangen erfordern, damit die gesamte Vorrichtung nicht wesentlich aufwendiger, länger und größer wird als die bisher für die konventionellen Pulver-Flüssigkeits-Polymethylmethacrylat-Knochenzemente üblichen Mischvorrichtungen. Es soll eine einfache Lösung gefunden werden, die es erlaubt, möglichst mit einer Auspressvorrichtung, die nur eine Stange und gegebenenfalls einen daran befestigten Teller aufweist, den Knochenzementteig auszutreiben.

Die Aufgaben der Erfindung werden gelöst durch eine Vorrichtung zum Lagern einer Monomerflüssigkeit und eines Zementpulvers als Ausgangskomponenten eines Knochenzementteigs sowie zum Mischen des Knochenzementteigs aus den Ausgangskomponenten und zum Austragen des gemischten Knochenzementteigs, die Vorrichtung aufweisend
einen röhrenförmigen Behälter, der an seiner Rückseite eine Aufnahme mit einem zylindrischen Innenraum bildet, in der ein Monomerflüssigkeitsbehälter angeordnet ist, wobei in dem Monomerflüssigkeitsbehälter die Monomerflüssigkeit enthalten ist, und der Behälter an seiner Vorderseite eine Kartusche mit einem zylindrischen Innenraum bildet, in der das Zementpulver enthalten ist, wobei
im Innenraum der Aufnahme ein in Längsrichtung der Aufnahme beweglicher Förderkolben angeordnet ist, der von einer Rückseite der Aufnahme aus zugänglich ist, wobei
im Innenraum der Kartusche ein in Längsrichtung im Inneren der Kartusche verschiebbarer Austragskolben zwischen dem Monomerflüssigkeitsbehälter und dem Zementpulver angeordnet ist, wobei
der Innenraum der Aufnahme und der Innenraum der Kartusche über eine für die Monomerflüssigkeit und für Gase durchlässige aber für das Zementpulver undurchlässige Verbindung miteinander verbunden sind, und wobei
der Förderkolben von der Rückseite aus mit einer Stange durchstechbar ist, wenn eine Bewegung des Förderkolbens in Richtung der Vorderseite des Behälters blockiert ist, wobei der Austragskolben durch ein weiteres Vortreiben der Stange durch den blockierten und durchstochenen Förderkolben hindurch in Richtung der Vorderseite der Kartusche vortreibbar ist.

Die Vorrichtung wird vorzugsweise für die Vertebroplastie oder Kyphoplastie eingesetzt. Es kann also vorgesehen sein, dass die Vorrichtung ein Knochenzement-Applikator zur Applikation des Knochenzementteigs im Bereich der Wirbelsäule ist, wobei bevorzugt der Knochenzement-Applikator eine verlängerte Austragsöffnung aufweist, die an der Vorderseite der Kartusche angeordnet ist, wobei besonders bevorzugt die verlängerte Austragsöffnung durch ein Austragsrohr, einen Schlauch und/oder einen Trokar realisiert ist.

Es kann bevorzugt vorgesehen sein, dass der Förderkolben für Flüssigkeiten und Gase undurchlässig ist und die Aufnahme an der Rückseite flüssigkeitsdicht und gasdicht abschließt.

Der Innenraum der Kartusche und der Innenraum der Aufnahme haben eine zylindrische Geometrie. Die zylindrische Form ist die einfachste, mit der sich der Innenraum der Kartusche und der Innenraum der Aufnahme realisieren lassen. Unter einer zylindrischen Form ist geometrisch die Form eines allgemeinen Zylinders mit einer beliebigen Grundfläche zu verstehen, also nicht nur ein Zylinder mit einer kreisförmigen Grundfläche. Die Innenwand des Innenraums der Kartusche und die Innenwand des Innenraums der Aufnahme können also durch den Zylindermantel eines Zylinders mit beliebiger Grundfläche realisiert sein, insbesondere mit unterschiedlicher Grundfläche sein, das heißt auch mit nicht kreisförmigen oder nicht runden Grundflächen. Erfindungsgemäß wird jedoch eine zylindrische Geometrie mit rotationssymmetrischer und insbesondere kreisrunder Grundfläche für beide Innenräume bevorzugt, da diese am einfachsten zu fertigen sind.

Die Stange ist vorzugsweise zylindrisch, kann aber eine abgerundete, angespitzte oder vorzugsweise eine stumpfe Spitze oder Kante aufweisen, um einerseits das Durchstechen des Förderkolbens zu erleichtern und andererseits den Austragskolben anzutreiben ohne diesen ebenfalls zu durchstechen oder derart stark zu verformen, dass er nicht mehr in der Kartusche in Richtung der Vorderseite bewegt werden kann.

Der Einfachheit halber wird im Rahmen der vorliegenden Erfindung der Knochenzementteig als gemischt angesehen, wenn die Monomerflüssigkeit das Zementpulver vollständig durchdrungen hat und dabei zu quellen beginnt. Bei einer vollständigen Durchmischung und zum Erreichen der Klebfreiheit kann es notwendig oder hilfreich sein, dass zusätzlich eine ausreichende Zeit verstreicht und/oder dass der Knochenzementteig zusätzlich mechanisch gemischt wird, zum Beispiel durch einen statischen Mischer. Insbesondere bei Vorrichtungen zur Anwendung in der Vertebroplastie führen jedoch die beim Auspressen des Knochenzementteigs durch die verlängerte und meist dünne Applikationsspitze (wie zum Beispiel einen Schlauch und einen Trokar) auftretenden Scherkräfte bereits zu einer vollständigen Mischung des Knochenzementteigs.

Dass der Förderkolben von einer Rückseite der Aufnahme aus zugänglich ist, bedeutet, dass eine Stange beziehungsweise ein Stößel einer Auspressvorrichtung, wie beispielsweise eine Kartuschenpistole, von hinten auf den Förderkolben drücken kann.

Es kann bevorzugt vorgesehen sein, dass die Vorrichtung derart ausgestaltet ist, insbesondere die Lagerung des Austragskolbens in dem Innenraum der Kartusche derart gestaltet ist, dass der Austragskolben durch ein weiteres Vortreiben der Stange durch den blockierten und durchstochenen Förderkolben hindurch in Richtung der Vorderseite der Kartusche vortreibbar ist.

Es kann ferner vorgesehen sein, dass die Vorrichtung eine Blockiereinrichtung aufweist, mit der der Förderkolben in dem Innenraum des Behälters blockierbar ist. Dabei kann vorzugsweise ein Anschlag als Blockiereinrichtung verwendet werden. Bevorzugt wird der Anschlag dadurch realisiert, dass der Innenraum der Kartusche einen kleineren Durchmesser beziehungsweise Querschnitt aufweist als der Innenraum der Aufnahme, so dass an dem Übergang von der Kartusche zur Aufnahme eine Abstufung entsteht, die als Blockiereinrichtung beziehungsweise als Anschlag für die Bewegung des Förderkolbens im Innenraum der Aufnahme in Richtung der Vorderseite des röhrenförmigen Behälters dient. Alternativ ist der Behälter, insbesondere die Innenwand der Aufnahme, mit der Blockiereinrichtung verformbar, so dass durch die Verformung der Förderkolben eingeklemmt wird oder die Verformung einen Anschlag bildet. Theoretisch kann die Blockiereinrichtung auch durch ein an der Rückseite des Förderkolbens in Richtung des äußeren Umfangs des Förderkolbens befestigtes Haltemittel, wie eine Schnur, ein Draht oder ein Stift, sein, die oder der an der Rückseite des Behälters am Behälter befestigt ist oder die oder der in den Behälter greift und so den Förderkolben im Behälter kippt und dadurch verkantet und blockiert.

Bevorzugt ist der Förderkolben im Bereich der Rückseite der Aufnahme oder in der Rückseite der Aufnahme angeordnet. Ebenso ist der Austragskolben vorzugsweise an der Rückseite der Kartusche oder im Bereich der Rückseite der Kartusche angeordnet.

Bei erfindungsgemäßen Vorrichtungen kann vorgesehen sein, dass der röhrenförmige Behälter einteilig ist, wobei vorzugsweise die Aufnahme und die Kartusche als ein einteiliger thermoplastischer Kunststoffkörper aufgebaut sind, wobei der Behälter besonders bevorzugt mit einem Spitzgussverfahren hergestellt ist.

Auf diese Weise kann der röhrenförmige Behälter kostengünstig gefertigt werden und ist besonders stabil auch am Übergang zwischen der Aufnahme und der Kartusche.

Ferner kann vorgesehen sein, dass die Querschnittsfläche des Innenraums der Kartusche kleiner ist als die Querschnittsfläche des Innenraums der Aufnahme.

Hiermit wird insbesondere erreicht, dass der zähe Knochenzementteig auch bei in Flussrichtung der Kartusche nachfolgendem Widerstand noch durch ein Austragsrohr mit einem statischen Mischer oder einen Schlauch mit einem angeschlossenen Trokar mit einer manuell angetriebenen Auspressvorrichtung auszupressen ist. Dadurch ist die Vorrichtung auch bei der Benutzung manuell angetriebener Auspressvorrichtung für die Vertebroplastie einsetzbar. Zudem kann die dadurch entstehende Abstufung als Anschlag für die Bewegung des Förderkolbens in Richtung der Vorderseite des röhrenförmigen Behälters verwendet werden.

Bevorzugt kann auch vorgesehen sein, dass an dem röhrenförmigen Behälter, insbesondere an der Rückseite der Aufnahme, ein Befestigungsmittel zur Befestigung einer Auspressvorrichtung angeordnet ist.

Dadurch lässt sich die Auspressvorrichtung mit der erfindungsgemäßen Vorrichtung schlüssig verbinden und gegen die Vorrichtung fixieren und dadurch die Monomerflüssigkeit gleichmäßig in das Zementpulver drücken und der Zementteig gleichmäßig aus der Kartusche austreiben.

Mit einer Weiterbildung der vorliegenden Erfindung wird vorgeschlagen, dass im Inneren des Behälters ein Anschlag vorgesehen ist, der die Bewegung des Förderkolbens in Richtung der Vorderseite begrenzt, wobei der Anschlag die Bewegung vorzugsweise derart begrenzt, dass der Förderkolben nicht vollständig aus der Aufnahme herauspressbar ist, wobei besonders bevorzugt der Anschlag als Abstufung durch eine unterschiedliche Form oder unterschiedliche Querschnitte des zylindrischen Innenraums der Kartusche und des zylindrischen Innenraums der Aufnahme am Übergang zwischen der Aufnahme und der Kartusche gebildet ist.

Mit dieser baulich einfachen und kostengünstigen Maßnahme wird die Position, an der der Förderkolben zuverlässig blockiert, festgelegt.

Damit wird zudem verhindert, dass beim Vortreiben des Förderkolbens dieser den Austragskolben in der Kartusche weit nach vorne treibt oder den Austragskolben zumindest derart weit nach vorne treibt, dass der Knochenzementteig aufgrund einer gemeinsamen Bewegung des Austragskolbens und des Förderkolbens beginnt aus der Kartusche auszufließen.

Dass der Anschlag die Bewegung des Förderkolbens in Richtung der Vorderseite des Behälters im Behälter begrenzt, bedeutet nicht zwangsläufig und automatisch, dass der Förderkolben an dem Anschlag anliegt, wenn er nicht mehr weiter in Richtung der Vorderseite getrieben werden kann. Zwischen dem Anschlag und dem Förderkolben können nämlich Reste oder Bruchstücke des Monomerflüssigkeitsbehälters, insbesondere Scherben einer Glasampulle oder Teile der Folie eines Folienbeutels als Monomerflüssigkeitsbehälter, eingeklemmt sein und dort verbleiben, ohne dass hierdurch die Funktionsfähigkeit der Vorrichtung in irgendeiner Weise eingeschränkt würde. Die Bewegung des Förderkolbens in Richtung der Vorderseite des Behälters ist blockiert, wenn der Förderkolben eine Position erreicht hat, in der seine Bewegung durch den Anschlag begrenzt ist. Durch die Blockade des Förderkolbens kann dieser von der Stange durchstochen werden. Damit definiert oder begrenzt der Anschlag die Position des Förderkolbens, in der der Förderkolben von der Stange durchstochen wird.

Bevorzugt kann vorgesehen sein, dass der Übergang von der Aufnahme zu der Kartusche als Anschlag zum Blockieren der Bewegung des Förderkolbens in Richtung der Vorderseite des Behälters ausgebildet ist.

Des Weiteren kann vorgesehen sein, dass die Verbindung im Austragskolben angeordnet ist und/oder in der Wandung des röhrenförmigen Behälters angeordnet ist, wobei vorzugsweise die Verbindung im Austragskolben angeordnet ist und die Vorderseite des Austragskolbens mit der dem Monomerflüssigkeitsbehälter zugewandten Rückseite des Austragskolbens verbindet.

Hiermit wird erreicht, dass die Monomerflüssigkeit leicht und ohne große Widerstände in die Kartusche geleitet beziehungsweise gedrückt werden kann. Zudem müssen so keine weiteren Leitungen in der Wandung des röhrenförmigen Behälters vorgesehen werden.

Gemäß einer bevorzugten Weiterbildung kann vorgesehen sein, dass an der Einmündung der Verbindung in den Innenraum der Kartusche ein für Gase und die Monomerflüssigkeit durchlässiger aber für das Zementpulver undurchlässiger Filter, insbesondere Porenfilter, angeordnet ist.

Hiermit wird verhindert, dass das Zementpulver in die Verbindung eindringt, dort mit der Monomerflüssigkeit reagiert und beim Anquellen des Zementpulvers in der Verbindung die Verbindung verschlossen wird.

Bevorzugt kann auch vorgesehen sein, dass das Zementpulver an der Vorderseite des Austragskolbens anliegt, insbesondere vollflächig anliegt, wobei vorzugsweise das Zementpulver in den Innenraum der Kartusche eingepresst ist.

Hierdurch wird verhindert, dass in der Kartusche größere Gaseinschlüsse verbleiben, die beim Mischen der Monomerflüssigkeit mit dem Zementpulver zu Gaseinschlüssen im Knochenzementteig führen könnten. Dies kann bei einem dicht geschütteten Zementpulver nicht passieren, da die Monomerflüssigkeit die Partikel des Zementpulvers gut benetzt und die Oberflächenspannung der Monomerflüssigkeit dann keine oder zumindest keine relevanten Gaseinschlüsse zwischen den Partikeln des Zementpulvers erlaubt.

Es kann dazu auch vorgesehen sein, dass das Volumen der Zwischenräume zwischen den Zementpartikeln des Zementpulvers Innenraum der Kartusche im Bereich von 22 Volumenprozent bis 40 Volumenprozent bezogen auf das Gesamtvolumen des Zementpulvers ist. Das Gesamtvolumen des Zementpulvers entspricht bevorzugt dem Volumen des Innenraums der Kartusche, der durch den Austragskolben und durch einen Verschluss in einer Austragsöffnung an der Vorderseite der Kartusche begrenzt ist.

Mit einer Weiterbildung der vorliegenden Erfindung wird vorgeschlagen, dass die Kartusche an der Vorderseite eine Austragsöffnung aufweist, die mit einem Verschluss verschlossen ist, wobei der Knochenzementteig durch die Austragsöffnung aus der Kartusche auspressbar ist, wenn die Austragsöffnung geöffnet ist, und wobei der Verschluss für Gase durchlässig und für das Zementpulver undurchlässig ist.

Dadurch kann die Kartusche gut zur Lagerung des Zementpulvers verwendet werden. Der Verschluss kann geöffnet werden. Der Innenraum der Kartusche und das Zementpulver können durch Evakuieren und Spülen des Innenraums der Kartusche mit einem sterilisierenden Gas, wie Ethylenoxid, durch den Verschluss hindurch sterilisiert werden.

Es kann dabei vorgesehen sein, dass der Verschluss durch den Vortrieb des Austragskolbens und den durch den Knochenzementteig auf den Verschluss vermittelten Druck aus der Austragsöffnung herausdrückbar ist.

Hiermit öffnet sich die Kartusche selbstständig beim Antreiben des Austragskolbens.

Es kann des Weiteren vorgesehen sein, dass an der Vorderseite der Kartusche ein Austragsrohr angeordnet und/oder ein flexibler Schlauch mit einem Trokar befestigt ist oder befestigbar ist, wobei der Knochenzementteig durch das Austragsrohr und/oder den flexiblen Schlauch und den Trokar auspressbar ist, wobei vorzugsweise an dem Austragsrohr oder dem Schlauch ein manuell verschließbares Ventilelement angeordnet ist, mit dem sich der Fluss des Knochenzementteigs steuern lässt.

Hiermit kann die Vorrichtung gut zur Applikation von Knochenzementteig an schwerer zugänglichen oder im Bereich von Röntgenstrahlung befindlichen Bereichen verwendet werden. Insbesondere kann die Vorrichtung so als Applikatorfür die Vertebroplastie verwendet werden.

Ferner wird mit der Erfindung auch vorgeschlagen, dass der Förderkolben von der Rückseite aus mit der Stange aufweisend eine Spitze oder eine Kante durchstechbar ist, wenn eine weitere Bewegung des Förderkolbens in Richtung der Vorderseite des röhrenförmigen Behälters blockiert ist.

Hiermit kann ein stabilerer Förderkolben verwendet werden, da der Druck, der zum Durchstechen des Förderkolbens notwendig ist, durch die geringere Auflagefläche der Spitze oder der Kante aufgrund der geringeren Anlagefläche bei gleicher Kraft vergrößert wird. Es versteht sich dabei, dass die Spitze oder Kante hierzu an der Vorderseite der Stange angeordnet ist, die an der Rückseite des Förderkolbens anliegt.

Bevorzugte Vorrichtungen können sich auch dadurch auszeichnen, dass der Förderkolben von der Rückseite aus mit der Stange mit einer Kraft von mindestens 1 kN durchstechbar ist, wenn eine weitere Bewegung des Förderkolbens in Richtung der Vorderseite des röhrenförmigen Behälters blockiert ist.

Hierdurch wird sichergestellt, dass der Förderkolben mit der Stange einer manuell angetriebenen Auspressvorrichtung durchstechbar ist.

Es kann auch vorgesehen sein, dass der Förderkolben an seiner Rückseite eine Auflagefläche für die Stange der Auspressvorrichtung aufweist und dass der Förderkolben aus einem Kunststoff gefertigt ist, insbesondere aus einem thermoplastischen Kunststoff gefertigt ist.

Damit ist der Förderkolben zunächst stabil anzutreiben und kann später, sobald der Förderkolben blockiert ist, mittig mit der Stange durchstochen werden. Dadurch ist die Vorrichtung stabil und sicher anwendbar. Es kann dabei vorgesehen sein, dass die Auflagefläche in der axialen Aufsicht mindestens gleich groß ist wie der Querschnitt der Stange.

Bevorzugt kann auch vorgesehen sein, dass der Förderkolben im Bereich der Angriffsfläche der Stange eine maximale Dicke von maximal 4 mm, bevorzugt von maximal 3 mm und ganz besonders bevorzugt von maximal 2 mm hat.

Hiermit wird sichergestellt, dass der Förderkolben mit einer manuell angetriebenen Auspressvorrichtung durchstechbar ist, da der Widerstand des Materials durch die geringe Dicke im zu durchstechenden Bereich reduziert wird.

Ferner kann vorgesehen sein, dass der Querschnitt des Innenraums der Kartusche maximal 4 cm² beträgt, bevorzugt maximal 2,5 cm² beträgt, besonders bevorzugt maximal 1,2 cm² beträgt.

Es kann analog auch vorgesehen sein, dass der Innendurchmesser der Kartusche kleiner als 20 mm ist, bevorzugt kleiner als 15 mm ist, besonders bevorzugt kleiner als 11 mm ist.

Durch den geringen Innendurchmesser ist der Querschnitt des Innenraums der Kartusche so klein, dass der zähe Knochenzementteig aus der Kartusche mit Hilfe einer manuellen Austragsvorrichtung ausgepresst werden kann, auch wenn noch weitere, die Strömung behindernde Leitungen, wie ein Schlauch, ein statischer Mischer oder ein Trokar, in Flussrichtung des Knochenzementteigs vorgesehen sind.

Des Weiteren kann vorgesehen sein, dass der Monomerflüssigkeitsbehälter eine Glasampulle, eine Plastikampulle, ein Kunststofffolienbeutel oder ein Aluminium-Kunststoff-Verbund-Beutel ist.

In diesen Monomerflüssigkeitsbehältern kann die Monomerflüssigkeit auch über längere Zeiträume in der Vorrichtung gelagert werden.

Es kann vorgesehen sein, dass das Volumen der Monomerflüssigkeit im Monomerflüssigkeitsbehälter mindestens so groß ist, wie das Volumen der mit Luft gefüllten Zwischenräume zwischen den Zementpartikeln des Zementpulvers im Innenraum der Kartusche.

Auch kann vorgesehen sein, dass das Volumen der Monomerflüssigkeit im Monomerflüssigkeitsbehälter mindestens so groß ist wie das Volumen der Flüssigkeitsleitungen zwischen dem Innenraum der Kartusche und dem Innenraum der Aufnahme plus das Volumen der mit Luft gefüllten Zwischenräume zwischen den Zementpulverpartikeln in der Kartusche.

Hierdurch kann sichergestellt werden, dass das gesamte Zementpulver von der Monomerflüssigkeit benetzt werden kann und so ein homogener Knochenzementteig erzeugt wird.

Gemäß einer bevorzugten Weiterbildung der vorliegenden Erfindung kann vorgesehen sein, dass in der Wandung der Aufnahme zumindest eine Belüftungsöffnung angeordnet ist, die den Innenraum der Aufnahme, in dem der Monomerflüssigkeitsbehälter angeordnet ist, mit der Umgebung verbindet, wobei bevorzugt die zumindest eine Belüftungsöffnung derart dicht im Bereich des Förderkolbens angeordnet ist, dass sie durch eine Bewegung des Förderkolbens in Richtung der Vorderseite der Aufnahme verschlossen wird, bevor der Monomerflüssigkeitsbehälter durch die Bewegung des Förderkolbens geöffnet ist.

Hierdurch kann der Innenraum der Aufnahme mit einem sterilisierenden Gas sterilisiert werden. Gleichzeitig kann die Monomerflüssigkeit nicht aus dem Innenraum der Aufnahme austreten, wenn die zumindest eine Belüftungsöffnung von dem sich in Richtung der Vorderseite der Aufnahme bewegenden Förderkolben verschlossen wird, bevor der Monomerflüssigkeitsbehälter durch die Bewegung des Förderkolbens geöffnet wird, also beispielsweise von dem Förderkolben im Innenraum der Aufnahme zerdrückt, zersplittert oder aufgerissen wird.

Zum Sicherstellen der Funktionsfähigkeit der erfindungsgemäßen Vorrichtung kann vorgesehen sein, dass an der Vorderseite der Aufnahme in der Verbindung zur Kartusche ein Sieb oder eine für Gase und Flüssigkeiten durchlässige poröse Scheibe vorgesehen ist.

Hierdurch kann verhindert werden, dass Bruchstücke oder abgetrennte Stücke des Monomerflüssigkeitsbehälters in die Kartusche und damit in den Knochenzement gelangen. Zudem kann so auch verhindert werden, dass solche Bruchstücke oder Stücke die flüssigkeitsdurchlässige Verbindung zwischen der Aufnahme und der Kartusche beeinträchtigen oder blockieren.

Gemäß einer bevorzugten Ausführungsform der vorliegenden Erfindung kann vorgesehen sein, dass ein von außen bedienbares Dreiwege-Ventil in der Flussrichtung des Knochenzementteigs in einer Leitung hinter der Kartusche angeordnet ist,
ein Auffangbehälter zum Aufnehmen von Knochenzementteig an dem Dreiwege-Ventil angeordnet ist, wobei Leitung in eine Applikationsöffnung mündet, die an dem von der Kartusche abgewandten Ende der Leitung angeordnet ist, wobei das Dreiwege-Ventil derart aufgebaut ist, dass es
in einer ersten Stellung eine Fluidverbindung zwischen der Applikationsöffnung und der Kartusche bereitstellt und einen Ablasskanal zum Auffangbehälter verschließt und
in einer zweiten Stellung eine Fluidverbindung zwischen der Applikationsöffnung und dem Auffangbehälter bereitstellt und einen Durchgang zur Kartusche verschließt.

Dadurch kann erreicht werden, dass bei einer Unterbrechung des Auspressvorgangs und durch Einstellen eines Dreiwege-Ventils, das mit dem Auffangbehälter, dem Schlauch und der Kartusche verbunden ist, der Druck von dem Knochenzement in einer verlängerten Austragsöffnung, wie einem Schlauch, genommen werden kann, ohne dass eine größere Menge des Knochenzementteigs nachfließt. Gleichzeitig kann auf diese Weise der Druck des Knochenzementteigs und der Ausgangskomponenten in der Kartusche bis zum Dreiwege-Ventil und insbesondere auch im Mischer, sofern vorhanden, aufrechterhalten werden. Dadurch ist die Zeit, die nach dem Öffnen des Dreiwege-Ventils bis zum erneuten Austritt des Knochenzementteigs vergeht, sehr kurz. Die Spannung der Kartusche wird zwischen dem Dreiwege-Ventil und dem Austragskolben also gehalten, wenn das Dreiwege-Ventil geschlossen ist, während eine schnelle Entspannung der verlängerten Austragsöffnung zwischen dem Dreiwege-Ventil und der Applikationsöffnung durch Abfließen des Knochenzementteigs durch das Dreiwege-Ventil in der geschlossenen Stellung erreicht wird. Damit der Knochenzementteig nicht die Umgebung oder den Anwender kontaminiert, ist der Auffangbehälter vorgesehen, der ein Abtropfen des durch das Dreiwege-Ventil austretenden Knochenzementteigs verhindert. Theoretisch kann es ausreichen, wenn der Knochenzementteig zurückgehalten wird. Der Auffangbehälter kann auch flexibel und/oder elastisch sein und sich beim Aufnehmen des aus dem Dreiwege-Ventil austretenden Knochenzementteigs ausdehnen.

Die Vorrichtung enthält so ein Notablassventil in Form des Dreiwege-Ventils, mit dem der Auspressvorgang sofort gestoppt werden kann, wenn der Knochenzementteig in unerwünschte Bereiche des Körpers, insbesondere des Wirbelkörpers zu fließen beginnt. Dieses Notablassventil druckentlastet die verlängerte Austragsöffnung der Vorrichtung, in dem der Nachdruck des Knochenzementteigs aus den Bereichen davor blockiert wird und gleichzeitig der vor dem Notablassventil befindliche Knochenzementteig druckentlastet wird, in dem ein Kanal in einen Auffangbehälter geöffnet wird, in den der Knochenzementteig austreten kann bis der Druck im Schlauch beziehungsweise im Trokar abgebaut ist.

Bei derartigen Vorrichtungen kann bevorzugt vorgesehen sein, dass der Auffangbehälter nach außen für den Knochenzementteig undurchlässig ist, vorzugsweise der Auffangbehälter flüssigkeitsdicht oder flüssigkeitsdicht und gasdicht ist. Es kann auch vorgesehen sein, dass der Auffangbehälter ein Volumen aufweist, das mindestens so groß ist wie die Hälfte des Volumens der verlängerten Austragsöffnung, insbesondere des Schlauchs und gegebenenfalls des Trokars, bevorzugt mindestens so groß ist wie das Volumen der verlängerten Austragsöffnung, insbesondere des Schlauchs und gegebenenfalls des Trokars.

Es kann erfindungsgemäß vorgesehen sein, dass zwischen der Kartusche und der verlängerten Austragsöffnung oder in der verlängerten Austragsöffnung oder zwischen der Kartusche und dem Dreiwege-Ventil ein Mischer zum Mischen des Knochenzements, insbesondere ein statischer Mischer, angeordnet ist, wobei vorzugsweise das Dreiwege-Ventil zwischen dem Mischer und der verlängerten Austragsöffnung angeordnet ist. In letzterem Fall kann vorgesehen sein, dass das Dreiwege-Ventil in der ersten Stellung eine Fluidverbindung zwischen der Applikationsöffnung und dem Mischer bereitstellt und in der zweiten Stellung den Durchgang zum Mischer verschließt.

Es kann bei erfindungsgemäßen Vorrichtungen mit Schlauch auch vorgesehen sein, dass der Schlauch zumindest bereichsweise flexibel ist. Es kann auch vorgesehen sein, dass die verlängerte Austragsöffnung in einem Anschluss mit einem Innengewinde, insbesondere in einem Luer-System-Adapter, oder in einem Trokar endet.

Des Weiteren kann vorgesehen sein, dass der Monomerflüssigkeitsbehälter im Inneren der Aufnahme durch eine Bewegung des Förderkolbens in Richtung der Vorderseite der Aufnahme zu öffnen ist, bevorzugt aufzubrechen oder aufzureißen ist.

Hierdurch wird erreicht, dass durch die axiale lineare Bewegung des Förderkolbens der Monomerflüssigkeitsbehälter geöffnet werden kann. Es kann dadurch eine Auspressvorrichtung mit nur einer Stange als axialer linearen Antrieb verwendet werden, um sowohl den Monomerflüssigkeitsbehälter zu öffnen als auch die Monomerflüssigkeit in die Kartusche zu pressen und auch den Knochenzementteig aus der Kartusche zu pressen.

Es kann auch vorgesehen sein, dass der Förderkolben an der rückseitigen Stirnseite eine Aufnahme oder eine Vertiefung, insbesondere eine zylindrische Vertiefung, für die Stange der Auspressvorrichtung aufweist, in der bevorzugt eine Gummimanschette als Dichtungselement angeordnet ist oder eine von der Stange durchstechbare, plastisch verformbare Kunststoff- oder Metallscheibe als Dichtungselement eingesetzt ist.

Hierdurch kann beim Auspressen des Zementteigs ein Austritt von Monomerflüssigkeit verhindert werden.

Es kann ferner vorgesehen sein, dass die Kartusche an der Vorderseite mit einem Kartuschenkopf abgeschlossen ist, wobei sich im Kartuschenkopf eine Austragsöffnung befindet und die Austragsöffnung mit einem für das Zementpulver in der Kartusche undurchlässigen und für Gas durchlässigen Verschluss, insbesondere einem Porenfilter, verschlossen ist, wobei vorzugsweise der Verschluss durch axiale Druckbeanspruchung oder durch manuelle Krafteinwirkung zu öffnen ist.

Des Weiteren kann vorgesehen sein, dass die Kartusche eine Druckfestigkeit größer 10 bar hat, bevorzugt größer 50 bar, besonders bevorzugt größer 70 bar hat.

Ferner kann vorgesehen sein, dass an einem Austragsrohr oder an einer Austragsöffnung an der Vorderseite der Kartusche ein Adapter zur Befestigung eines Schlauchs oder eines Trokars angeordnet ist.

Dadurch ist die Vorrichtung für die Vertebroplastie einsetzbar.

Dabei kann vorgesehen sein, dass der Schlauch einen Innendurchmesser kleiner 4 mm hat und eine Mindestdruckfestigkeit von 10 bar.

Die der vorliegenden Erfindung zugrundeliegenden Aufgaben werden auch gelöst durch eine Auspressvorrichtung zum Vortreiben eines Förderkolbens und eines Austragskolbens einer Vorrichtung zum Mischen eines Knochenzementteigs aus Ausgangskomponenten und zum Austragen des gemischten Knochenzementteigs, die Auspressvorrichtung aufweisend eine Halterung zur Befestigung der Vorrichtung und eine gegen die Halterung axial vortreibbare Stange, bei der die Stange an der Vorderseite eine harte Spitze oder Kante zum Durchstechen des blockierten Förderkolbens der Vorrichtung aufweist, wobei auf der Spitze oder Kante eine abnehmbare Kappe mit einer ebenen Vorderseite angeordnet oder aufsetzbar ist.

Die Spitze oder Kante kann stumpf, abgeflacht oder abgerundet sein, um den von der Kante oder der Spitze ausgeübten Druck einstellen zu können, der einerseits mindestens notwendig ist, um den Förderkolben der Vorrichtung durchstechen zu können, und der gleichzeitig nicht so hoch sein soll, dass der Austragskolben durchstochen oder derart stark verformt wird, dass er in der Kartusche blockiert.

Dabei kann vorgesehen sein, dass die Auspressvorrichtung zum Vortreiben des Förderkolbens und des Austragskolbens und zum Durchstechen des blockierten Förderkolbens einer erfindungsgemäßen Vorrichtung vorgesehen ist, wobei bevorzugt die Vorrichtung in die Auspressvorrichtung eingesetzt ist oder in einer gemeinsamen Verpackung mit der Auspressvorrichtung verpackt ist.

Die erfindungsgemäße Auspressvorrichtung ist besonders vorteilhaft mit einer erfindungsgemäßen Vorrichtung einsetzbar.

Ferner kann vorgesehen sein, dass die Auspressvorrichtung einen gegen die Halterung manuell schwenkbaren Hebel aufweist, wobei die Stange manuell mit dem Hebel mit Hilfe einer Klemmeinrichtung, wie Klemmbacken oder Klemmscheiben, vortreibbar ist, wobei sich die Klemmeinrichtung bei einer Bewegung des Hebels in einer Richtung fest mit der Stange verbindet und bei einer Bewegung des Hebels in die andere Richtung von der Stange löst, so dass die Stange mit dem Hebel nur in einer Richtung bewegbar ist.

Hierdurch kann die Auspressvorrichtung in einer Hand gehalten und gleichzeitig mit der gleichen Hand der Hebel bedient werden, um die Stange anzutreiben, wobei der Vortrieb der Stange zum Vortreiben eines Kolbens bei Anwendung mit der Kappe auf der Spitze oder Kante der Stange als auch zum Durchstechen eines Kolbens bei der Anwendung der Stange ohne die Kappe verwendet werden kann.

Die der vorliegenden Erfindung zugrundeliegenden Aufgaben werden auch gelöst durch ein Verfahren zur Herstellung eines Knochenzementteigs, insbesondere eines pastenförmigen Polymethylmethacrylat-Knochenzementteigs, wobei der Knochenzementteig aus einem Zementpulver und einer Monomerflüssigkeit mit einer Vorrichtung hergestellt wird, wobei die Vorrichtung röhrenförmigen Behälter aufweist, der an seiner Rückseite eine Aufnahme mit einem zylindrischen Innenraum bildet, in der ein Monomerflüssigkeitsbehälter mit der Monomerflüssigkeit darin angeordnet ist, und der Behälter an seiner Vorderseite eine Kartusche mit einem zylindrischen Innenraum bildet, in der das Zementpulver enthalten ist, gekennzeichnet durch die folgenden nacheinander ablaufenden Schritte:
a) Einsetzen der Vorrichtung in eine Auspressvorrichtung, die Auspressvorrichtung aufweisend eine axial vortreibbare Stange,
b) ein Förderkolben, der innerhalb der Aufnahme an deren Rückseite beweglich gelagert ist, wird mit der Stange in Richtung der Kartusche vorgetrieben, wobei durch die Bewegung des Förderkolbens der Monomerflüssigkeitsbehälter geöffnet wird und die Monomerflüssigkeit aus der Aufnahme in die Kartusche gepresst wird, wobei im Innenraum der Kartusche sich das Zementpulver mit der Monomerflüssigkeit mischt,
c) die Bewegung des Förderkolbens in Richtung der Vorderseite des Behälters wird blockiert,
d) die Stange sticht durch den Förderkolben und trifft nach dem Durchstechen des Förderkolbens auf einen Austragskolben, der innerhalb der Kartusche beweglich gelagert ist,
e) der Austragskolben wird mit der Stange in Richtung der Vorderseite der Kartusche vorgetrieben, während die Stange durch den blockierten und durchstochenen Förderkolben hindurch läuft, wobei durch die Bewegung des Austragskolbens die Mischung aus dem Zementpulver und der Monomerflüssigkeit aus der Kartusche als Knochenzementteig ausgetrieben wird.

In Schritt a) wird vorzugsweise die Auspressvorrichtung an der Aufnahme der Vorrichtung befestigt.

Es kann auch vorgesehen sein, dass in Schritt b) der Monomerflüssigkeitsbehälter zunächst zwischen dem Förderkolben und dem Austragskolben komprimiert wird bevor er geöffnet wird.

Es kann ferner vorgesehen sein, dass in Schritt b) der Monomerflüssigkeitsbehälter in Splitter zerbricht oder aufgerissen wird oder aufgeschnitten wird.

Des Weiteren kann in Schritt b) vorgesehen sein, dass, nachdem die Monomerflüssigkeit in die Kartusche eingepresst wurde, die Zementpulverpartikel in der Monomerflüssigkeit anquellen und die radikalische Polymerisation der Monomerflüssigkeit durch Reaktion des Beschleunigers mit dem Initiator ausgelöst wird.

Es kann auch vorgesehen sein, dass in Schritt e) die Stange durch die Bruchstücke, Fetzen oder Reste des Monomerflüssigkeitsbehälters läuft, während der Austragskolben mit der Stange vorangetrieben wird.

Es kann vorgesehen sein, dass das Verfahren mit einer erfindungsgemäßen Vorrichtung umgesetzt wird.

Das Verfahren lässt sich besonders gut mit einer erfindungsgemäßen Vorrichtung umsetzen.

Ferner kann vorgesehen sein, dass das Verfahren mit einer erfindungsgemäßen Auspressvorrichtung umgesetzt wird.

Die erfindungsgemäße Auspressvorrichtung ist besonders gut zum Umsetzen des erfindungsgemäßen Verfahrens geeignet.

Des Weiteren kann vorgesehen sein, dass die Stange an der Vorderseite eine harte Spitze oder Kante zum Durchstechen des blockierten Förderkolbens der Vorrichtung aufweist, wobei in Schritt a) auf der Spitze oder Kante eine abnehmbare Kappe mit einer ebenen Vorderseite angeordnet ist und die Kappe nach Schritt c) von der Spitze oder Kante entfernt wird und die Stange mit der Spitze oder der Kante in den Förderkolben getrieben wird, wobei vorzugsweise zuvor die Vorrichtung aus der Auspressvorrichtung entnommen wird und die Vorrichtung nach Entfernen der Kappe von der Spitze oder Kante wieder in die Auspressvorrichtung eingesetzt wird.

Hiermit kann zunächst der Förderkolben stabil vorangetrieben werden und später der Förderkolben mit der Spitze oder Kante der Stange ohne zu großen Kraftaufwand durchstochen werden.

Gemäß einer bevorzugten Weiterbildung des erfindungsgemäßen Verfahrens kann vorgesehen sein, dass vor Schritt c) oder in Schritt c) durch den auf die Mischung des Zementpulvers mit der Monomerflüssigkeit wirkenden Druck ein Verschluss, insbesondere ein Porenfilter, in einer Austragsöffnung an der Vorderseite der Kartusche bewegt oder hervorgedrückt wird, wobei hierauf vorzugsweise der Verschluss aus der Austragsöffnung entfernt wird und ein verlängertes Austragsrohr oder ein Schlauch mit einem Trokar an der Vorderseite der Kartusche befestigt wird, und/oder die Kappe von der Spitze oder der Kante der Stange entfernt wird.

Hierdurch ist es für den Anwender anhand des hervortretenden Verschlusses erkennbar, dass die Vorrichtung einsatzbereit ist und der Knochenzementteig aus der Vorrichtung ausgepresst werden kann.

Bevorzugt kann auch vorgesehen sein, dass der zerbrochene oder aufgeschlitzte oder aufgeplatzte Monomerflüssigkeitsbehälter in Schritt b) zusammengeschoben wird und gleichzeitig Gas aus der Aufnahme durch eine Verbindung in die Kartusche gedrückt wird und durch das Zementpulver in der Kartusche nach außen gedrückt wird und wobei in Schritt d) die durch den Förderkolben getriebene Stange die Bruchstücke des Monomerflüssigkeitsbehälters verdrängt.

Hierdurch wird erreicht, dass die Reste des Monomerflüssigkeitsbehälters in dem von dem Förderkolben zusammengeschobenen Bereich der Aufnahme zusammengeschoben werden und so ein Großteil der Monomerflüssigkeit aus der Aufnahme in die Kartusche gepresst wird.

Es kann vorgesehen sein, dass in Schritt e) ein gasdurchlässiger und pulverundurchlässiger Verschluss aus einer Austragsöffnung an der Vorderseite der Kartusche durch den Druck des vorangegangenen Knochenzementteigs gedrückt wird oder der Verschluss manuell entfernt wird.

Hierdurch sieht der Anwender, dass die Vorrichtung bereit zur Applikation des Knochenzementteigs ist.

Schließlich kann vorgesehen sein, dass in Schritt d) unmittelbar vor der Applikation des Knochenzementteigs an der Vorderseite der Kartusche an einer Austragsöffnung ein Trokar mit einem Schlauch mit der Kartusche verbunden wird und der Knochenzementteig anschließend durch den Schlauch und den Trokar und gegebenenfalls ein Austragsrohr infolge der Vorwärtsbewegung des Austragskolbens durch weitere Betätigung der Auspressvorrichtung ausgepresst wird.

Dadurch kann der Knochenzementteig auch an schwer zugänglichen Regionen, wie beispielsweise im Bereich von Wirbeln, und in Röntgenstrahlung appliziert werden.

Der Erfindung liegt die überraschende Erkenntnis zugrunde, dass es durch den zunächst beweglichen und anschließend blockierten und dadurch von einer Stange einer Auspressvorrichtung durchstechbaren Förderkolben gelingt, die Stange zunächst zum Antreiben des Förderkolbens zu nutzen, um den Monomerflüssigkeitsbehälter zu öffnen und um die Monomerflüssigkeit in das Zementpulver zu transferieren, und anschließend die gleiche Stange zum Antreiben eines in Reihe dahinter angeordneten Austragskolbens zu nutzen, um den Knochenzementteig aus der Kartusche auszupressen. Zum Durchstechen des Förderkolbens kann dabei erstens: eine Kappe mit ebener Vorderseite von einer spitzen oder kantigen Vorderseite der Stange entfernt werden oder - äquivalent dazu - eine Kappe mit einer Spitze oder einer Kante kann auf die Stange mit ebener Vorderseite aufgesetzt werden, um damit den Förderkolben zu durchstechen, oder zweitens: die Spitze der Stange kann direkt zum Durchstechen und zum Antreiben des Förderkolbens geeignet sein. Für den letzteren Fall muss der Förderkolben ausreichen stabil und/oder ausreichend leichtgängig sein, um den Förderkolben zunächst zum Öffnen und gegebenenfalls Zerdrücken des Monomerflüssigkeitsbehälters und zum Auspressen der Monomerflüssigkeit aus der Aufnahme in die Kartusche zu nutzen, und gleichzeitig ausreichend instabil beziehungsweise durchstechbar sein, um den Förderkolben, wenn dessen Bewegung blockiert ist, mit der Vorderseite der Stange durchstechen zu können. Hierzu sind Folienbeutel als Monomerflüssigkeitsbehälter bevorzugt, da diese der Bewegung des Förderkolbens weniger Widerstand entgegensetzen als die Splitter zerbrechender Glasampullen.

Durch die Verwendung eines vom Förderkolben separaten und unabhängigen Austragskolbens kann auch eine Kartusche mit einem besonders schmalen Innenraum verwendet werden, der kleiner ist als die Aufnahme für die üblichen und breiteren Monomerflüssigkeitsbehälter. Hierdurch kann der zähe und hochviskose Knochenzementteig auch gegen den Widerstand eines Schlauchs noch aus dem dünnen Innenraum der Kartusche ausgepresst und appliziert werden. Dadurch sind die Vorrichtung und das Verfahren auch für die Vertebroplastie einsetzbar.

Die erfindungsgemäße Vorrichtung gemäß Anspruch 1 hat die wesentlichen Vorteile, dass die beiden Ausgangskomponenten des Knochenzements, insbesondere des Spine-Zements, im geschlossenen Zementiersystem gelagert sind und dass die Vermischung der Ausgangskomponenten in der geschlossenen Vorrichtung erfolgt. Das bedeutet, dass die Vorrichtung nicht vom Anwender befüllt werden muss. Es handelt sich um ein Full-Prepacked-Zementiersystem. Der medizinische Anwender hat keinerlei Kontakt mit den einzelnen Ausgangskomponenten der Spine-Zemente beziehungsweise der Knochenzemente. Die Geruchsbelästigung ist dadurch nur noch minimal. Ein besonderer Vorteil der Vorrichtung besteht auch darin, dass durch das einfache Vorwärtsbewegen einer Stange einer manuell angetriebenen Auspressvorrichtung die Monomerflüssigkeit in das Zementpulver gepresst wird. Dabei wird die zwischen den Zementpulverpartikeln vorhandene Luft durch die Monomerflüssigkeit verdrängt. Es entsteht ein homogener Knochenzementteig, ohne dass ein manuelles Mischen mit Mischstäben mit Mischflügeln notwendig ist. Das bedeutet, dass das fehlerbehaftete manuelle Mischen nicht mehr notwendig ist. Die Bedienung der Vorrichtung ist maximal vereinfacht. Es handelt sich um ein Ready-to-use-System.

Die Augmentation von frakturierten Wirbelkörpern erfolgt immer unter ständiger Fluoroskopie (Röntgenkontrolle). Ein weiterer Vorteil der Vorrichtung besteht darin, dass auf Grund des geringen Querschnitts der Kartusche 1 der Auspressdruck auf den Zementteig bei der Betätigung der manuell angetriebenen Auspressvorrichtung so hoch ist, dass der Zementteig durch einen druckfesten Schlauch von 20 cm bei einem Innendurchmesser von 3 mm gepresst werden kann. Infolge des 20 cm langen Schlauchs sind die Hände des Radiologen bzw. des Orthopäden außerhalb des Bereichs der Röntgenstrahlen. Die Strahlenbelastung der Ärzte wird dadurch deutlich vermindert.

Durch geeigneten Aufbau der Vorrichtung gemäß Anspruch 16 beziehungsweise durch geeignete Verfahrensschritte kann erreicht werden, dass eine manuell angetriebene Auspressvorrichtung einsetzbar ist. Ferner wird durch geeignete erfindungsgemäße Maßnahmen erreicht, dass beim Lagern und Mischen des Knochenzementteigs keine Monomerflüssigkeit und kein Knochenzementteig aus der Vorrichtung austritt und die Umgebung kontaminiert.

Der besondere Vorteil der erfindungsgemäßen Vorrichtung besteht darin, dass mit manuell angetriebenen Auspressvorrichtungen ein Zweikomponenten-Spine-Zement beziehungsweise der Knochenzementteig für die Vertebroplastie über einen dünnen Schlauch in einen Trokar ausgepresst werden kann. Die Augmentation von Wirbelkörpern erfolgt unter permanenter Röntgenkontrolle. Der Schlauch zwischen dem Trokar und dem Applikator ermöglicht es, dass der Arzt mit seinen Händen nicht im Bereich der Röntgenstrahlung arbeitet. Es sind dabei keine komplexen, teuren Hydraulik-Applikationsvorrichtungen notwendig.

Die Vorrichtung kann als hygienisches Wegwerfprodukt verwendet werden, da sie sehr weitgehend aus Kunststoff gefertigt werden kann und weil alle Teile einschließlich der Innenräume und des Zementpulvers mit Hilfe von Ethylenoxid sterilisierbar sind.

Eine beispielhafte erfindungsgemäße Vorrichtung zum Lagern, Vermischen und Austragen von Polymethylmethacrylat-Knochenzement für die Augmentation von Wirbelkörpern kann beispielsweise aufweisen:
a) einen einteiligen röhrenförmigen Körper, der in einem hohlzylinderförmigen Kartuschenabschnitt 1 (der Kartusche) und einem hohlzylinderförmigen Kartuschenabschnitt 2 (der Aufnahme) gegliedert ist, wobei der Innendurchmesser der hohlzylinderförmigen Kartusche kleiner ist als der Innendurchmesser der hohlzylinderförmigen Aufnahme, und wobei am Ende der Aufnahme ein Verbindungselement zur Verbindung mit einer Auspressvorrichtung angebracht ist,
b) einem Monomerflüssigkeitsbehälter in der Aufnahme,
c) einem für Flüssigkeiten und Gase undurchlässigen, im Hohlraum der Aufnahme axial beweglichen Förderkolben,
d) Zementpulver, das in der Kartusche angeordnet ist,
e) einem axial im Hohlraum der Kartusche beweglichen Austragskolben, der das Zementpulver in Richtung der Aufnahme begrenzt, wobei der Austragskolben mindestens eine für Gase und Flüssigkeiten durchlässige, jedoch für Pulverpartikel undurchlässige Durchführung besitzt, welche die Vorderseite und die Rückseite des Austragskolbens verbindet,
f) einem Kartuschenkopf, der das Zementpulver in der Kartusche begrenzt, wobei der Kartuschenkopf eine Durchführung als Austragsöffnung enthält, die mit einem Austragsrohr verbunden ist,
g) einem für Gase und Flüssigkeiten durchlässigen Verschlusskolben, der die Durchführung des Kartuschenkopfs reversibel verschließt, und
h) wobei der Förderkolben mit einer Stange (die auch als Stößel bezeichnet werden kann) einer Auspressvorrichtung bei einer Krafteinwirkung von größer 1 kN durchstoßen wird.

Die Vorrichtung kann als hygienisches Wegwerfprodukt verwendet werden, da sie sehr weitgehend aus Kunststoff gefertigt werden kann und weil alle Teile einschließlich der Innenräume und des Zementpulvers mit Hilfe von Ethylenoxid sterilisierbar sind.

Ein erfindungsgemäßes Verfahren kann beispielsweise mit der beispielhaften Vorrichtung zur Vermischung des Zementpulvers mit der Monomerflüssigkeit unter Bildung von Knochenzementteig mit den aufeinanderfolgenden Schritten umgesetzt werden:
a) Verbinden der Auspressvorrichtung mit dem Verbindungselement der Aufnahme der Kartusche,
b) Vortreiben der Stange der Auspressvorrichtung,
c) Verschieben des Förderkolbens in Richtung des Kartuschenkopfs,
d) Komprimierung des mindestens einen Monomerflüssigkeitsbehälters zwischen dem Austragskolben und dem Förderkolben,
e) Bersten oder Reißen des Monomerflüssigkeitsbehälters,
f) Zusammenschieben des geborstenen oder gerissenen Monomerflüssigkeitsbehälters und Auspressen der Luft aus dem Innenraum der Aufnahme und der Monomerflüssigkeit mit dem Förderkolben durch die mindestens eine Öffnung des Austragskolbens in das Zementpulver in den Innenraum der Kartusche,
g) Ausbreitung der Monomerflüssigkeit in dem Zementpulver unter gleichzeitiger Verdrängung der Luft aus den Zwischenräumen der Zementpulverpartikel,
h) Benetzung der Zementpulverpartikel mit der Monomerflüssigkeit,
i) Entweichen der Luft aus dem Zementpulver durch den gasdurchlässigen Verschlusskolben,
j) Anquellen der Zementpulverpartikel durch die Monomerflüssigkeit und Auslösung der radikalischen Polymerisation der Monomerflüssigkeit durch Reaktion des Beschleunigers mit dem Initiator,
k) Bildung des Knochenzementteigs aus dem Zementpulver und der Monomerflüssigkeit,
l) Abtrennen der Auspressvorrichtung von der Kartusche,
m) Entfernen eines Tellers beziehungsweise einer aufgesteckten Kappe von der Stange aus Metall der Auspressvorrichtung,
n) Gegebenenfalls erneutes Verbinden der Auspressvorrichtung mit der Aufnahme,
o) weitere Bewegung der Metallstange in Richtung Kartuschenkopf,
p) Durchstoßen des Förderkolbens an der Auflagefläche durch die Stange,
q) Verdrängung des zerborstenen Monomerflüssigkeitsbehälters durch die Metallstange,
r) Bewegung des Austragskolbens in Richtung des Kartuschenkopfs durch die Metallstange, wobei die Metallstange sich durch den durchstoßenen Förderkolben und die Reste des zerborstenen Monomerflüssigkeitsbehälters bewegt,
s) Austreiben des Verschlusskolbens oder manuelle Entfernung des Verschlusskolbens,
t) Verbinden eines Adapters des Austragsrohrs mit einem Trokar oder mit einem Schlauch,
u) Auspressen des Knochenzementteigs durch das Austragsrohr infolge der Vorwärtsbewegung des Austragskolbens durch Betätigung der Auspressvorrichtung.

Im Folgenden werden weitere Ausführungsbeispiele der Erfindung anhand von dreizehn schematisch dargestellten Figuren erläutert, ohne jedoch dabei die Erfindung zu beschränken. Dabei zeigt:
Figur 1: eine schematische Querschnittansicht einer beispielhaften erfindungsgemäßen Vorrichtung zum Lagern und Mischen einer Monomerflüssigkeit und eines Zementpulvers;
Figur 2: eine schematische Seitenansicht der Vorrichtung nach Figur 1;
Figur 3: vier schematische Querschnittansichten der Vorrichtung nach den Figuren 1 und 2 mit einer angeschlossenen erfindungsgemäßen Auspressvorrichtung übereinander zur Verdeutlichung des Ablaufs eines erfindungsgemäßen Verfahrens;
Figur 4: eine schematische Querschnittansicht durch den vorderen Teil der erfindungsgemäßen Vorrichtung nach den Figuren 1 bis 3 mit vorgeschobenem Porenfilter;
Figur 5: drei schematische perspektivische Ansichten auf erfindungsgemäße Vorrichtungen nach den Figuren 1 bis 4 mit unterschiedlichen Anschlüssen am Kartuschenkopf;
Figur 6: eine schematische Querschnittansicht als Detail-Ansicht eines Dreiwege-Ventils für eine verlängerte Austragsöffnung nach Figur 3 unten;
Figur 7: eine schematische Querschnittansicht als Detail-Ansicht des Dreiwege-Ventils nach Figur 6 mit einer Schnittebene senkrecht zur Flussrichtung des Knochenzementteigs;
Figur 8: eine schematische Querschnittansicht als Detail-Ansicht der Verbindung der Vorrichtung nach den Figuren 1 bis 7 mit einer erfindungsgemäßen Auspressvorrichtung;
Figur 9: eine schematische Querschnittansicht als Detail-Ansicht des Übergangs von der Aufnahme zur Kartusche vor dem Austrag des Knochenzementteigs;
Figur 10: eine schematische Querschnittansicht als Detail-Ansicht des mittleren Teils der Vorrichtung nach den Figuren 1 bis 9 während der Knochenzementteig ausgepresst wird;
Figur 11: eine schematische perspektivische Teilansicht als Detail-Ansicht auf den rückseitigen Teil der Vorrichtung nach den Figuren 1 bis 10 vor dem Einsetzen in die Auspressvorrichtung;
Figur 12: zwei schematische perspektivische Querschnittansichten durch ein Ventilsystem für eine alternative erfindungsgemäße Vorrichtung und zwar ein Dreiwege-Ventil in geschlossener Position beziehungsweise Stellung (Figur 12 oben) und in geöffneter Position beziehungsweise Stellung (Figur 12 unten);
Figur 13: zwei schematische Querschnittansichten in Aufsicht durch das Ventilsystem nach Figur 12 und zwar das Dreiwege-Ventil in geöffneter Position beziehungsweise Stellung (Figur 13 unten) und in geschlossener Position beziehungsweise Stellung (Figur 13 oben); und
Figur 14: eine schematische Querschnittansicht durch ein drittes erfindungsgemäßes Ausführungsbeispiel für eine Vorrichtung zum Lagern und Mischen einer Monomerflüssigkeit und eines Zementpulvers.

In den Figuren werden der Einfachheit halber teilweise bei gleichen Bauteilen die gleichen Bezugszeichen auch für unterschiedliche Ausführungsformen verwendet.

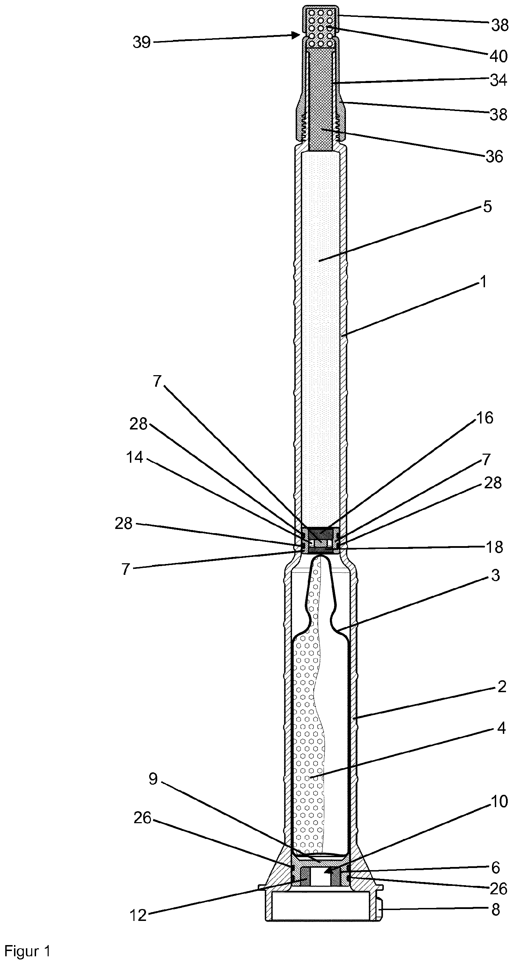

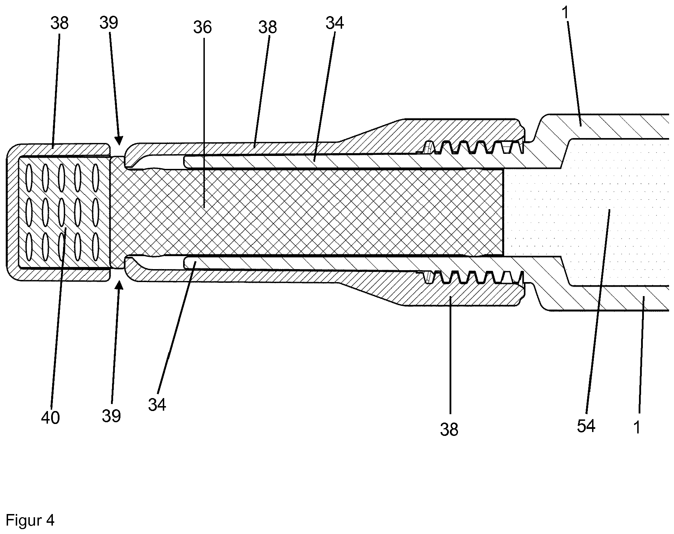

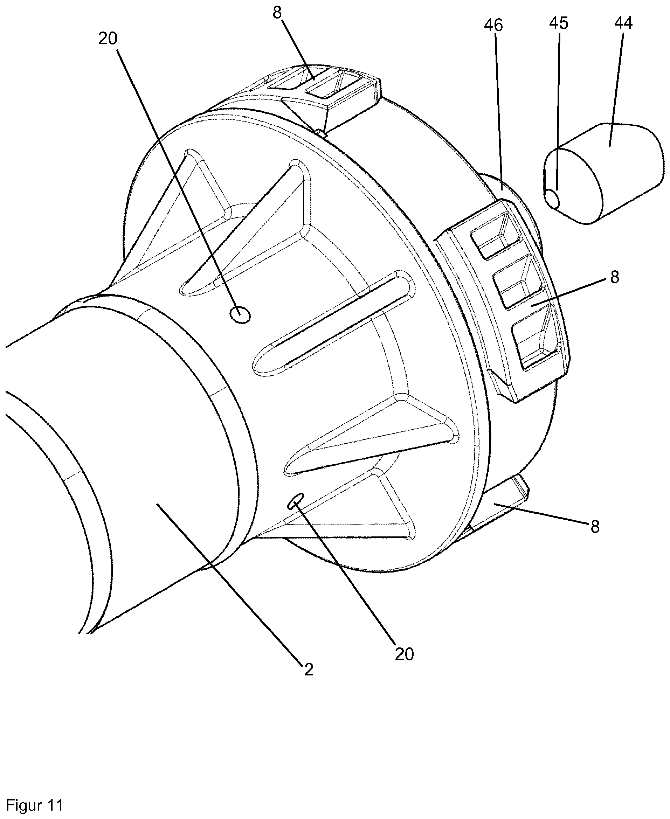

In den Figuren 1 bis 11 sind Abbildungen einer ersten erfindungsgemäßen Vorrichtung gezeigt. Die Figuren 1 bis 3 und 5 zeigen verschiedene schematische Ansichten einer ersten beispielhaften erfindungsgemäßen Vorrichtung. Die Figuren 4, 6 bis 10 zeigen schematische Querschnittansichten als Detailansichten durch verschiedene Bereiche der erfindungsgemäßen Vorrichtung sowie Figur 11 eine perspektivische Teilansicht der Rückseite der Vorrichtung.

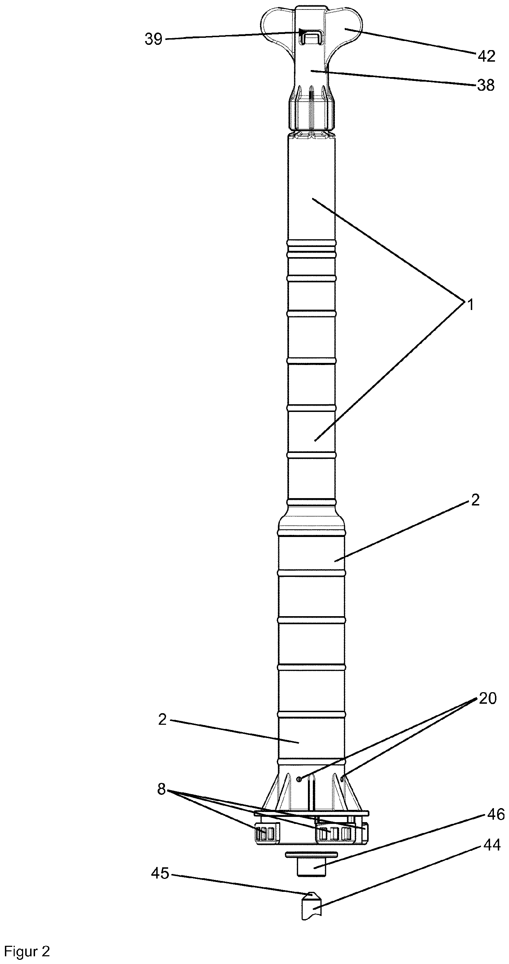

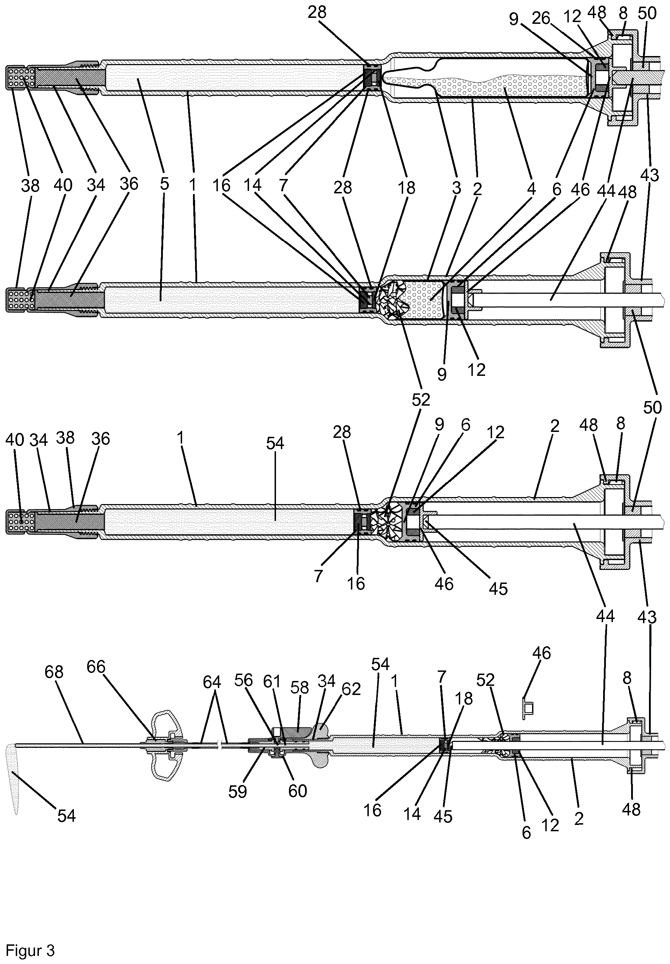

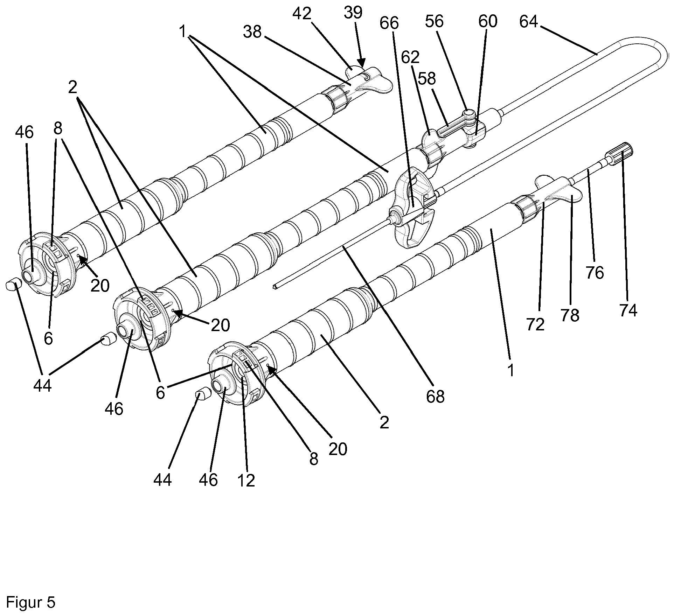

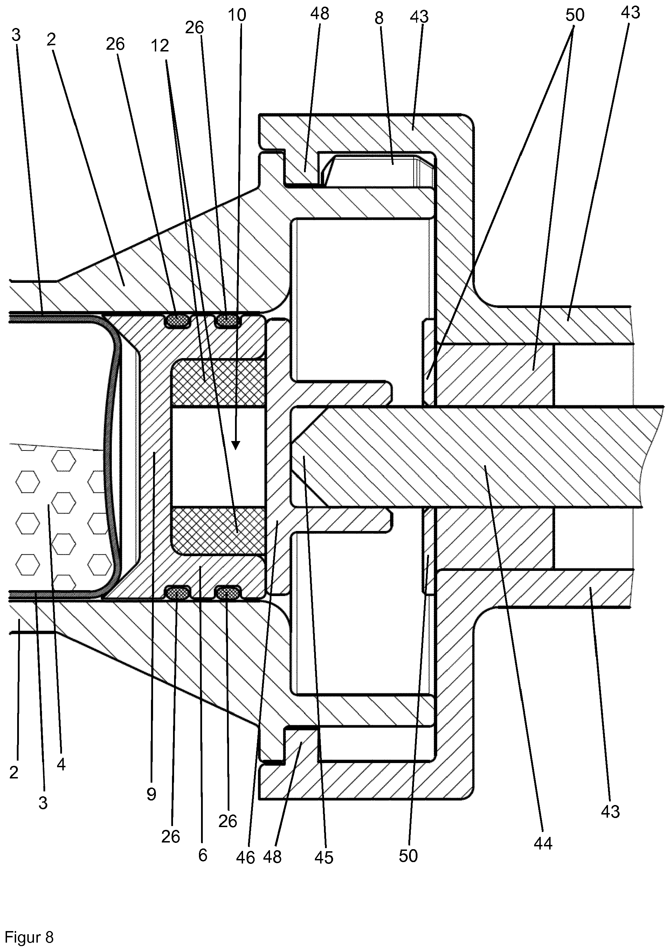

Die erfindungsgemäße Vorrichtung besteht im Wesentlichen aus einem röhrenförmigen Behälter aus Kunststoff, der als vorderen Teil (in den Figuren 1 und 2 oben, in den Figuren 3, 4, 6, 9 und 10 links und in Figur 5 rechts oben) eine Kartusche 1 mit einem zylindrischen Innenraum bildet und der als hinteren Teil eine Aufnahme 2 für eine Glasampulle 3 (oder Kunststoffampulle 3) bildet. Die Rückseite der Vorrichtung ist in den Figuren 1 und 2 unten, in den Abbildungen der Figur 3 rechts und in Figur 5 unten links sowie in Figur 8 und 11 gezeigt. Die Röhrenform ist besonders gut in den Querschnittansichten der Figuren 1 und 3 zu erkennen. Sowohl der Innenraum der Kartusche 1 als auch der Innenraum der Aufnahme 2 sind zylindrisch mit kreisförmiger Grundfläche. Dabei ist der Durchmesser des Innenraums der Kartusche 1 kleiner als der Durchmesser des Innenraums der Aufnahme 2. Der Behälter mit der Aufnahme 2 und der Kartusche 1 wird vorzugsweise mit Hilfe einer Spritzgusstechnik aus Kunststoff hergestellt. Die Aufnahme 2 weist also ebenfalls einen zylindrischen Innenraum auf, in den die Glasampulle 3 eingesteckt ist. In der Glasampulle 3 befindet sich die Monomerflüssigkeit 4. In den Innenraum der Kartusche 1 ist ein Zementpulver 5 eingefüllt oder vorzugsweise eingepresst. Die Monomerflüssigkeit 4 und das Zementpulver 5 bilden die Ausgangskomponenten 4, 5 für einen PMMA-Knochenzement, der mit der Vorrichtung hergestellt werden kann. Aufgrund der Glasampulle 3 kann die Monomerflüssigkeit 4 sehr lange in der Aufnahme 2 und dadurch in der Vorrichtung gelagert werden. Das Zementpulver 5 kann ebenfalls über längere Zeiträume in der Vorrichtung gelagert werden. Die Vorrichtung ist damit zum Lagern der Monomerflüssigkeit 4 und des Zementpulvers 5 als Ausgangskomponenten eines Knochenzementteigs des PMMA-Knochenzements geeignet. Die Vorrichtung ist aber auch zum Mischen des Knochenzementteigs aus den Ausgangskomponenten und zum Austragen des gemischten Knochenzementteigs geeignet und vorgesehen.

In der Aufnahme 2 ist ein im zylindrischen Innenraum der Aufnahme 2 in Längsrichtung beweglicher Förderkolben 6 aus Kunststoff angeordnet. Der Förderkolben 6 ist im Bereich der Rückseite der Aufnahme 2 angeordnet. Die Glasampulle 3 kann mit dem Förderkolben 6 in der Aufnahme 2 zusammengedrückt und dabei zersplittert werden, indem der Förderkolben 6 in Richtung der Vorderseite, also in Richtung der Kartusche 1 gedrückt wird. Der Förderkolben 6 weist an der Vorderseite Abstreifer auf, mit denen Splitter der Glasampulle 3 von der Innenwand der Aufnahme 2 abgestreift werden.

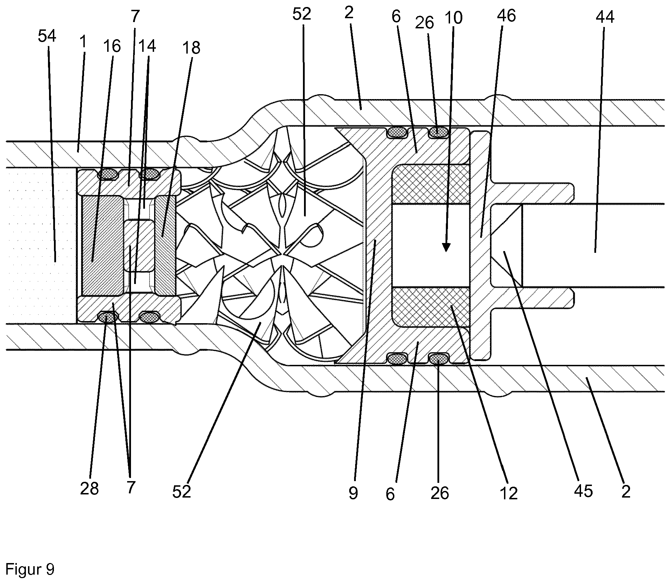

In dem Innenraum der Kartusche 1 ist in dessen Rückseite (in den Figuren 1 und 2 Richtung unten, in den Figuren 3 und 9 Richtung rechts) ein Austragskolben 7 aus Kunststoff angeordnet. An der Rückseite der Aufnahme 2 ist ein Befestigungsmittel 8 vorgesehen, mit dem die Aufnahme 2 an einer Auspressvorrichtung 43 (in Figur 1 nicht zu sehen, siehe aber Figuren 3 und 8) verbunden werden kann. Das Befestigungsmittel 8 ist vorzugsweise zur Bildung eines Bajonettverschlusses 8 geeignet und vorgesehen. Dadurch kann der Förderkolben 6, der von der Rückseite der Aufnahme 2 aus frei zugänglich ist, mit der Auspressvorrichtung 43 in Richtung der Vorderseite vorgetrieben werden.

Der Förderkolben 6 ist wie ein kurzes Rohr geformt, das an der seiner Vorderseite, die der Glasampulle 3 zugewandt ist, mit einer ebenen Wandung abgeschlossen ist. Die Wandung weist in der Mitte eine Sollbruchstelle 9 auf, die von der Rückseite des Förderkolbens 6 aus durch eine Vertiefung 10 von hinten zugänglich ist. Die Stabilisierung und Abdichtung des restlichen Förderkolbens 6 wird mit einer röhrenförmigen Hülse 12 erreicht, die ebenfalls aus Kunststoff gefertigt ist und vorzugsweise als Gummimanschette 12 ausgeführt ist. Im Bereich der Sollbruchstelle 9 weist der Förderkolben 6 also eine verminderte Materialstärke auf.

Die Kartusche 1 und die Aufnahme 2 sind einteilig als gemeinsames Kunststoffteil ausgeführt. Die Aufnahme 2 und die Kartusche 1 sind für die Monomerflüssigkeit 4 flüssigkeitsdurchlässig miteinander über eine Durchführung 14 im Austragskolben 7 verbunden. Die Durchführung 14 durch den Austragskolben 7 mündet durch einen für das Zementpulver 5 undurchlässigen aber für die Monomerflüssigkeit 4 durchlässigen Porenfilter 16 in den Innenraum der Kartusche 1.

In der Verbindung zur Durchführung 14 ist in dem röhrenförmigen Behälter ein Filter 18 angeordnet, mit dem die Splitter der Glasampulle 3 zurückgehalten werden können. Statt dem Filter 18 oder zusätzlich zum Filter 18 kann auch ein Sieb vorgesehen sein. Der Filter 18 ist in dem Austragskolben 7 angeordnet.

Mehrere Belüftungsöffnungen 20 sind in der Wandung der Aufnahme 2 vorgesehen, durch die der Innenraum der Aufnahme 2 mit Hilfe eines sterilisierenden Gases wie Ethylenoxid sterilisiert werden kann. Diese Belüftungsöffnungen 20 sind in den Figuren 1 und 3 nicht zu sehen, aber in den Figuren 2, 5 und 11 dargestellt und bezeichnet. Die Belüftungsöffnungen 20 sind unmittelbar benachbart zum Förderkolben 6 angeordnet, so dass der Förderkolben 6 die Belüftungsöffnungen 20 unmittelbar verschließt, wenn er in Richtung der Kartusche 1 vorgetrieben wird. Dadurch wird verhindert, dass Monomerflüssigkeit 4 durch die Belüftungsöffnungen 20 austreten kann, wenn die Glasampulle 3 in der Aufnahme 2 geöffnet wurde.

Der zylindrische Förderkolben 6 hat einen zur Zylindergeometrie des Innenraums der Aufnahme 2 passenden Außenumfang und ist über zwei umlaufende Dichtungen 26 gegen die Innenwand der Aufnahme 2 flüssigkeitsdicht abgedichtet. Ebenso ist der Austragskolben 7 über zwei umlaufende Dichtungen 28 gegen die Innenwand der Kartusche 1 flüssigkeitsdicht abgedichtet. Diese Dichtungen 26, 28 dienen dazu, einen Austritt von Monomerflüssigkeit 4 oder von Knochenzement zu verhindern, um eine Kontamination der Umgebung (des OP-Saals und des Anwenders) zu verhindern. Die Dichtungen 26, 28 können hierzu aus Gummi bestehen.

Die Vorderseite der Kartusche 1 mündet in ein Austragsrohr 34, das ein Außengewinde aufweist. Im Inneren der Austragsrohrs 34 ist ein Porenfilter 36 angeordnet, der für das Zementpulver 5 undurchlässig aber für Gase durchlässig ist. An dem Außengewinde des Austragsrohrs 34 ist eine Kappe 38 befestigt, wobei der vordere Teil der Kappe 38 mit einem Styropor oder Schaumstoff 40 gefüllt ist. An der Kappe 38 sind zwei Flügel 42 vorgesehen, so dass die Kappe 38 nach Art einer Flügelschraube bequem von dem Austragsrohr 34 abgeschraubt werden kann. Die Kappe 38 weist seitliche Öffnungen 39 auf. Durch diesen Aufbau kann das Innere der Kartusche 1 und das Zementpulver 5 mit Hilfe von Ethylenoxid sterilisiert werden, da die Öffnungen 39 in der Kappe 38, das Styropor oder der Schaumstoff 40, der Porenfilter 36 und die Zwischenräume zwischen den Pulverpartikeln des Zementpulvers 5 luftdurchlässig sind. Gleichzeitig kann Luft aus der Aufnahme 2 durch das Zementpulver 5, den Porenfilter 36, das Styropor oder der Schaumstoff 40 und die Öffnungen 39 in der Kappe 38 herausgepresst werden, wenn der Förderkolben 6 in Richtung der Aufnahme 2 gepresst wird.

Das Zementpulver 5 ist in der Kartusche 1 eingeschlossen, da alle Öffnungen 39 und Durchgänge 14 mit Hilfe der Porenfilter 16, 36 für das Zementpulver 5 undurchlässig verschlossen sind. Der Inhalt der Kartusche 1 kann dabei durch Evakuieren und Spülen mit Ethylenoxid sterilisiert werden. Dadurch ist die Vorrichtung auch zum langfristigen Lagern des Zementpulvers 5 geeignet.

Figur 3 zeigt vier schematische Querschnittansichten der erfindungsgemäßen Vorrichtung nach den Figuren 1 und 2 übereinander zur Verdeutlichung des Ablaufs eines erfindungsgemäßen Verfahrens. Zu Beginn des Verfahrens liegt die Vorrichtung im Ausgangszustand vor, so wie sie auch in Figur 1 gezeigt ist. In diesem Zustand wird die Vorrichtung in eine erfindungsgemäße Auspressvorrichtung 43 eingesetzt, die weitgehend einer herkömmlichen Kartuschenpistole entspricht. Die Auspressvorrichtung 43 umfasst eine linear vortreibbare Stange 44. Von der Auspressvorrichtung 43 ist nur der vordere Teil dargestellt in der sie sich von herkömmlichen Auspressvorrichtungen unterscheidet. Die Auspressvorrichtung 43 umfasst auch einen Griff und einen Kipphebel (in Figur 3 nicht zu sehen) zum manuellen Antreiben der Stange 44 der Auspressvorrichtung 43, wie bei herkömmlichen manuell angetriebenen Auspressvorrichtungen auch. Die Vorrichtung wird mit dem Befestigungsmittel 8 an der Auspressvorrichtung 43 befestigt (siehe oberste Abbildung in Figur 3 sowie Figur 8). Die Stange 44 endet an ihrer Vorderseite in einer Spitze 45, die zunächst mit einer abnehmbaren Kappe 46 abgedeckt ist. Die Stange 44 drückt mit der Kappe 46 auf die Hülse 12 beziehungsweise Gummimanschette 12 des Förderkolbens 6, wenn die Stange 44 von der Auspressvorrichtung 43 in die Aufnahme 2 hineingedrückt wird. Die Auspressvorrichtung 43 ist hierzu über ein Gegenbefestigungsmittel 48 an die Rückseite der Aufnahme 2 angeschlossen, so dass die Kappe 46 beim Vortreiben der Stange 44 auf den Förderkolben 6 drückt und diesen in Richtung der Kartusche 1 vortreibt. Hierzu ist die Stange 44 gegen ein Lager 50 und darüber gegen das Gegenbefestigungsmittel 48 und damit gegen die Aufnahme 2 linear beweglich gelagert.

Die Auspressvorrichtung 43 wird bedient und dabei die Stange 44 und mit der Stange 44 der Förderkolben 6 in Richtung der Kartusche 1 vorgetrieben. Da die Glasampulle 3 an der Vorderseite an dem Austragskolben 7 anliegt, verkleinert sich der Innenraum der Aufnahme 2 und die Glasampulle 3 zerbricht und die Monomerflüssigkeit 4 tritt aus der Glasampulle 3 in den Innenraum der Aufnahme 2 aus. Der Austragskolben 7 kann nicht von der Glasampulle 3 in Richtung des Porenfilters 36 geschoben werden, wenn das Zementpulver 5 trocken ist, also nicht von der Monomerflüssigkeit 4 benetzt ist, da das trockene Zementpulver 5 nicht fließfähig ist und eine Bewegung des Austragskolbens 7 blockiert. Diese Situation ist in Figur 3, 2. Abbildung von oben sowie in einer vergrößerten Ausschnittansicht in Figur 8 gezeigt. Überstehende Luft aus der Aufnahme 2 wird durch den Filter 18, die Durchführung 14, den Porenfilter 16, durch die Zwischenräume zwischen den Partikeln des Zementpulvers 5, durch den Porenfilter 36, durch den Schaumstoff 40 und aus den Öffnungen 39 in der Kappe 38 aus der Vorrichtung herausgedrückt.

Von der Glasampulle 3 bleiben schließlich nur kleine Splitter 52 zurück, die von dem Filter 18 zurückgehalten werden und in dem röhrenförmigen Behälter verbleiben. Die Monomerflüssigkeit 4 wird durch den Filter 18, die Durchführung 14 und den Porenfilter 16 in das Zementpulver 5 gepresst und beginnt dort mit dem Zementpulver 5 zu reagieren, so dass sich aus dem Gemisch 54 der Knochenzementteig 54 bildet. Diese Situation ist in Figur 3, dritte Abbildung von oben gezeigt sowie als Detailansicht in Figur 9. Der Porenfilter 36 wird, sobald das Gemisch 54 entstanden ist, von dem auf das Gemisch 54 aufgrund des Drucks auf den Austragskolben 7 wirkenden Druck nach vorne getrieben und komprimiert den Schaumstoff 40. Wenn der Porenfilter 36 nun nach vorne rutscht, so wird er von außen für den Anwender durch die Öffnung 39 in der Kappe 38 sichtbar. Diese Situation ist in Figur 4 gezeigt. Hierzu hat der Porenfilter 36 vorzugsweise eine andere Farbe und Helligkeit als der Schaumstoff 40. Beispielsweise kann der Schaumstoff 40 weiß sein und der Porenfilter 36 rot.

In diesem Zustand wird die Vorrichtung aus der Auspressvorrichtung 43 entnommen und die Kappe 46 von der Spitze 45 der Stange 44 entfernt. Alternativ kann die Spitze 45 der Stange 44 auch einfach mit einer hierfür ausreichenden Kraft durch den Förderkolben 6 hindurchgetrieben werden. Zudem wird die Kappe 38 mit dem Porenfilter 36 und dem Schaumstoff 40 abgeschraubt und stattdessen eine verlängerte Austragsöffnung auf das Austragsrohr 34 aufgeschraubt (siehe auch Figur 5). Anschließend wird die Vorrichtung mit der verlängerten Austragsöffnung wieder über das Befestigungsmittel 8 an der Auspressvorrichtung 43 befestigt.

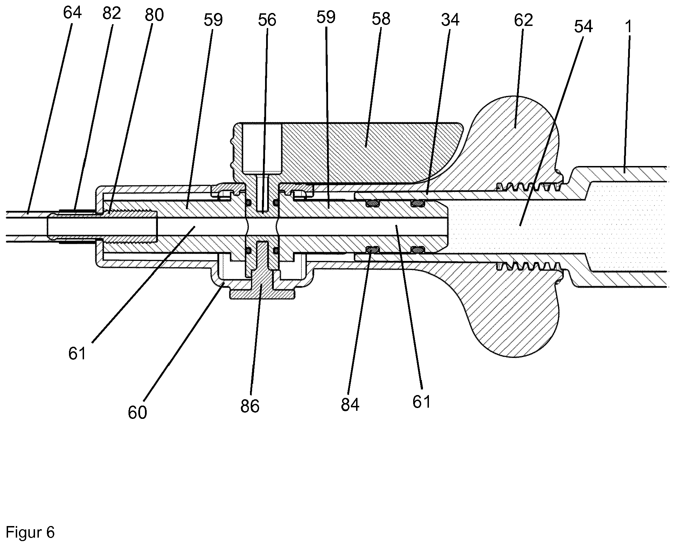

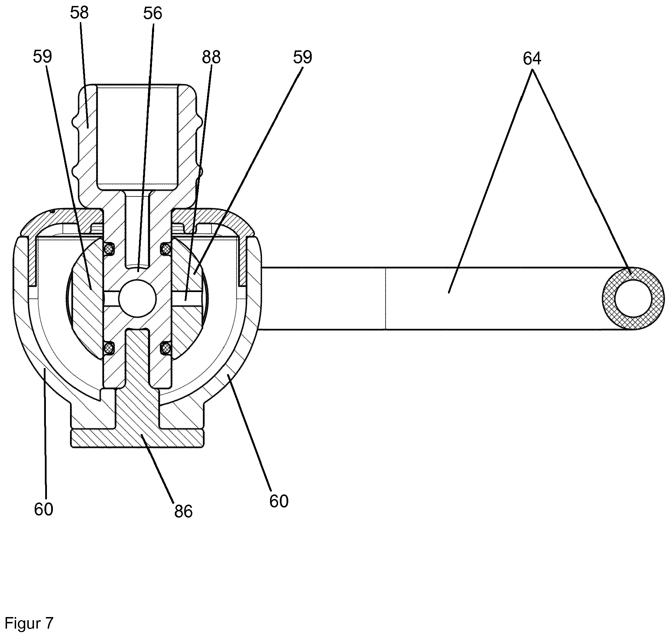

Die verlängerte Austragsöffnung umfasst ein Ventil-System mit einem Dreiwege-Ventil 56, das von außen über einen Knebelgriff 58 manuell bedient werden kann. Das Dreiwege-Ventil 56 sitzt in dichter Passung in einem Rohr 59, das einen Ventilsitz 59 bildet. Die Figuren 6 und 7 zeigen schematische Querschnittansichten als Detailansichten durch ein Ventilsystem, das als Teil der erfindungsgemäßen Vorrichtung nach den Figuren 1 bis 5 verwendet werden kann. Im Bereich des Dreiwege-Ventils 56 ist ein nach außen geschlossener Auffangbehälter 60 zur Aufnahme von Knochenzementteig 54 angeordnet. In dem Rohr 59 ist ein Durchgang 61 vorgesehen, der das Innere des Rohrs 59 bildet. Der Durchgang 61 in der Vorderseite des Rohrs 59 kann über das Dreiwege-Ventil 56 mit der Aufnahme 60 verbunden werden, so dass unter Druck stehender Knochenzementteig 54 von der vorderen Seite der verlängerten Austragsöffnung nach hierhin ausweichen kann und der Knochenzementteig 54 nur wenig aus der verlängerten Austragsöffnung nachfließt, wenn das Dreiwege-Ventil 56 in dieser geschlossenen Stellung ist. Hierdurch wird die Menge an nachströmendem Knochenzementteig 54 reduziert, wenn das Dreiwege-Ventil 56 verwendet wird. Das Dreiwege-Ventil 56 wird mit Hilfe von Flügeln 62 auf das Austragsrohr 34 aufgeschraubt. Der vordere Teil des Rohrs 59 ist mit einem Schlauch 64 verbunden, der in einen Trokar 66 mündet.

Durch weiteres Vortreiben der Stange 44 wird der Förderkolben 6 an der Sollbruchstelle von der Spitze 45 und der Stange 44 durchstochen. Der Förderkolben 6 kann dabei nicht ausweichen, da der Übergang zwischen der Aufnahme 2 und der Kartusche 1 im röhrenförmigen Behälter als Abstufung ausgebildet ist, die einen Anschlag für die Beweglichkeit des Förderkolbens 6 in Richtung des Austragsrohrs 34 bildet. Dabei können die Splitter 52 der Glas- oder Kunststoffampulle 3 zwischen dem Förderkolben 6 und der Abstufung eingeklemmt werden beziehungsweise sein. Die Stange 44 drückt sich mit der Spitze 45 auch durch restliche Aufnahme 2 der Vorrichtung, in der sich die Splittern 52 befinden. Schließlich trifft die Stange 44 auf den Austragskolben 7 und drückt diesen In Richtung des Austragsrohrs 34.

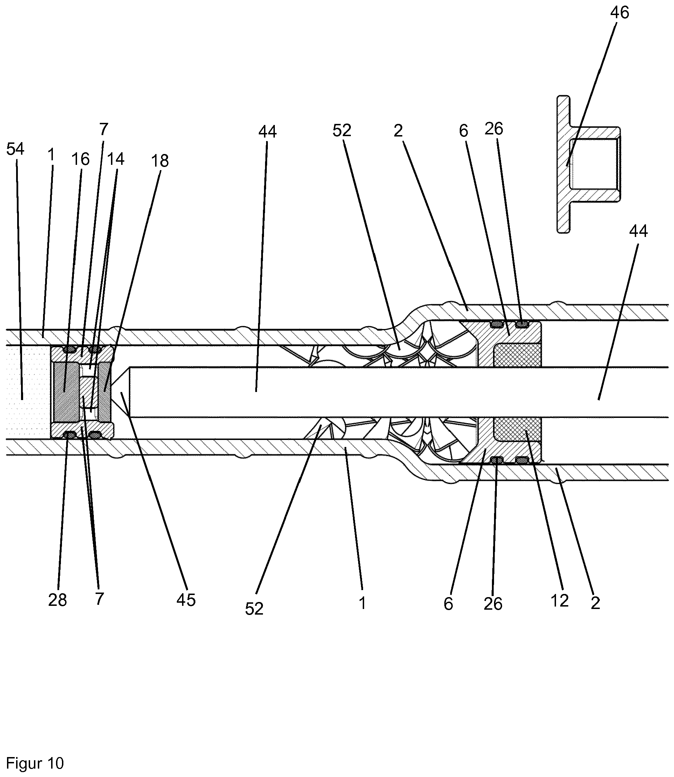

Über den Schlauch 64 und den Trokar 66 kann der Knochenzementteig 54 ausgetragen werden. Dazu wird der Austragskolben 7 mit der Stange 44 in Richtung des Austragsrohrs 34 vorgetrieben (siehe hierzu auch die Detailansicht nach Figur 10). Der Knochenzementteig 54 aus dem Inneren der Kartusche 1 wird bei geöffnetem Dreiwege-Ventil 56, so wie es in Figur 3 (unterste Abbildung) und in den Figuren 6 und 7 gezeigt ist, durch das Austragsrohr 34, durch das Dreiwege-Ventil 56 und den Durchgang 61, durch den Schlauch 64 und den Trokar 66 und durch eine Trokarspitze 68 des Trokars 66 ausgetrieben und kann dort an den Wirbeln eines Patienten appliziert werden oder theoretisch zur weiteren Verarbeitung verwendet werden. Zur Unterbrechung des Flusses des Knochenzementteigs 54 kann das Dreiwege-Ventil 56 bedient werden. Unter Druck stehender Knochenzementteig 54 aus dem Schlauch 64 und dem Trokar 66 kann durch das Dreiwege-Ventil 56 in den Auffangbehälter 60 abfließen, ohne die Umgebung zu kontaminieren. Dadurch wird der Fluss des Knochenzementteigs 54 schnell unterbrochen.

Der Trokar 66 ist direkt mit dem Schlauch 64 verbunden, kann aber auch über einen Adapter mit dem Schlauch 64 verbunden werden. In Figur 5 ist bei der ersten Ausführung von unten rechts eine Variante gezeigt, bei der ein Anschluss 72 einen Luer-System-Adapter 74 über einen kurzen Schlauch 76 mit der Kartusche 1 verbindet. Der Anschluss 72 kann mit Hilfe von Flügeln 78 nach Art einer Flügelschraube auf das Austragsrohr 34 der Kartusche 1 geschraubt werden. Hierzu weist der Anschluss 72 ein passendes Innengewinde auf.

In der Detailansicht der Ausschnittvergrößerung nach Figur 6 des Ventilsystems ist zusätzlich zu sehen, dass das Rohr 59 mit einem Einsatz 80, der einen mit dem Durchgang 61 fluchtenden Kanal aufweist, mit dem Schlauch 64 verbunden ist. Dazu ist der Einsatz 80 in das Rohr 59 eingeschraubt. Um eine druckdichte Verbindung zu gewährleisten, ist der Schlauch 64 mit einer Metallhülse 82 auf den Einsatz 80 gekrimpt und das Rohr 59 mit zwei umlaufenden Dichtungen 84 gegen die Innenwand des Austragsrohrs 34 abgedichtet. Die Unterseite des Dreiwege-Ventils 56 ist mit einem Stopfen 86 abgesichert, so dass das Dreiwege-Ventil 56 nicht einfach aus dem Ventilsitz 59 beziehungsweise dem Rohr 59 herausgezogen werden kann. In der Schnittebene nach Figur 7, die zu der Schnittebene nach Figur 6 senkrecht liegt und die senkrecht zum Durchgang 61 liegt, ist ein Ablasskanal 88 im Ventilsitz 59 zu erkennen, durch die bei geschlossener Stellung des Dreiwege-Ventils 56 der Knochenzementteig 54 von der vorderen Seite der verlängerten Austragsöffnung in den Auffangbehälter 60 abfließen kann.

Die Öffnungen 39 dienen auch als visuelle Marker anhand derer festgestellt werden kann, ob die Vorrichtung einsatzbereit ist. Wenn der Porenfilter 36 aufgrund des Drucks des Knochenzementteigs 54 nach vorne gedrückt wird und dabei das Styropor 40 in der Kappe 38 zusammenpresst, wird der Porenfilter 36 durch die Öffnungen 39 sichtbar. Dadurch kann der Anwender erkennen, dass der Knochenzementteig 54 fertig gemischt in der Kartusche 1 vorliegt und damit einsatzbereit ist. Der Anwender kann zu diesem Zeitpunkt die Kappe 46 entfernen, die Auspressvorrichtung 43 wieder mit der Vorrichtung verbinden und den Förderkolben 6 an der Sollbruchstelle 9 mit der Spitze 45 der Stange 44 durchstoßen und so den Austragskolben 7 antreiben und dadurch den Knochenzementteig 54 aus der Kartusche 1 austreiben. Zudem kann auch am Außengewinde des Austragsrohrs 34 ein geeigneter Aufbau, wie beispielsweise der Trokar 66 mit dem Schlauch 64 oder der Schlauch 76 mit dem Luer-System-Adapter 74, angeschlossen werden.

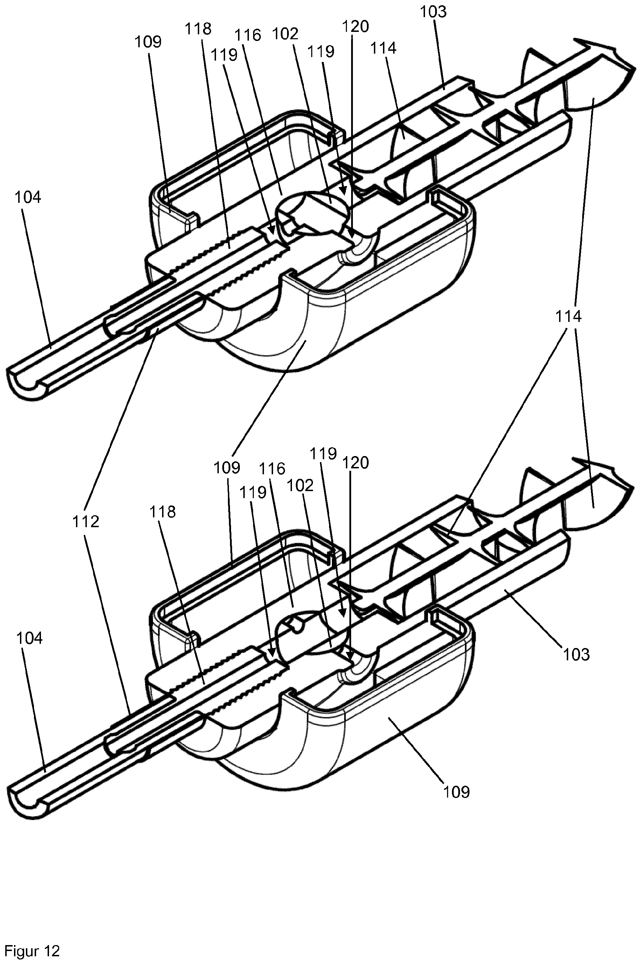

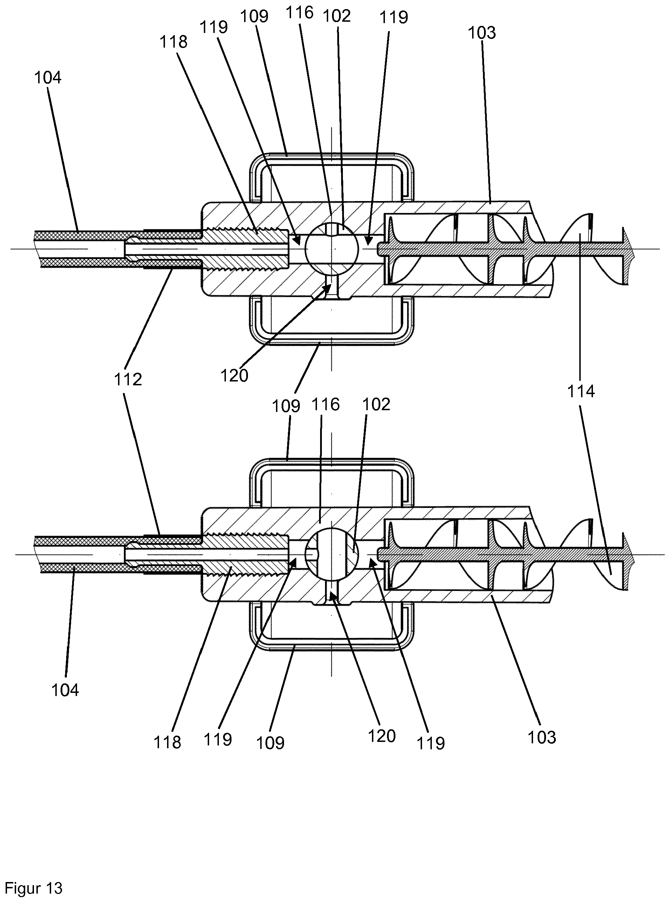

Die Figuren 12 und 13 zeigen jeweils zwei schematische perspektivische Querschnittansichten durch ein beispielhaftes Dreiwege-Ventil 102 für eine zweite alternative erfindungsgemäße Vorrichtung und zwar das Dreiwege-Ventil 102 in geschlossener Position beziehungsweise Stellung (Figur 12 oben und Figur 13 unten) und in geöffneter Position beziehungsweise Stellung (Figur 12 unten und Figur 13 oben), um die Funktionsweise des Dreiwege-Ventils 102 anhand des inneren Aufbaus zu erläutern.

Der Aufbau der alternativen zweiten erfindungsgemäßen Vorrichtung gleicht dort, wo es nicht anders beschrieben oder in den Figuren 12 und 13 zu sehen ist, dem vorherigen ersten Ausführungsbeispiel nach den Figuren 1 bis 11.

Ein Rohr 103 ist als verlängerte Austragsöffnung an der Vorderseite einer Kartusche (in den Figuren 12 und 13 nicht zu sehen, aber wie nach dem vorherigen Ausführungsbeispiel ausgeführt) angeordnet. Hinter dem Ventilsystem ist ein Schlauch 104 an dem Ventilsystem befestigt, durch den der Knochenzementteig (in den Figuren 12 und 13 nicht dargestellt) ausgetragen werden kann. Damit der Knochenzementteig nicht unkontrolliert austreten kann ist analog der Ausführung nach den Figuren 6 und 7 ein Auffangbehälter 109 vorgesehen, der zur Aufnahme von aus dem Dreiwege-Ventil 102 austretendem Knochenzement vorgesehen ist. Dadurch wird eine Kontamination der Umgebung - also insbesondere des OP-Bereichs - mit Knochenzementteig verhindert. Der Schlauch 104 ist mit einer Hülse 112 aus Metall über einen Krimp-Anschluss durchdicht mit dem Ventilsystem verbunden.

Im Inneren des Rohrs 103 befindet sich ein statischer Mischer 114, der sich bis an das Dreiwege-Ventil 102 heran erstreckt. Mit Hilfe des statischen Mischers 114 werden die Ausgangskomponenten des Knochenzements beziehungsweise der vorgemischte Knochenzementteig durchmischt, wenn diese durch den statischen Mischer 114 im Rohr 103 gepresst werden.

Das drehbare Dreiwege-Ventil 102 ist in den Querschnittansichten nach den Figuren 12 und 13 in der Symmetrieebene der darin zu erkennenden Kanäle geschnitten. Die Kanäle sind also zylindrisch und setzen sich in den weggeschnittenen Teil des Dreiwege-Ventils 102 spiegelsymmetrisch fort. Die Kanäle bilden im Dreiwege-Ventil 102 ein T-Stück. Das Dreiwege-Ventil 102 sitzt in einem passenden Ventilsitz 116, der dicht an dem Dreiwege-Ventil 102 anliegt und so die Kanäle abdichtet, wenn diese in dem Ventilsitz 116 gedreht sind. Im Ventilsitz 116 befinden sich zwei Durchgänge 119, durch die der größere, durchgehende Kanal im Dreiwege-Ventil 102 mit dem Rohr 103 auf der einen Seite und mit einem Einsatz 118 aus Metall zum Befestigen des Schlauchs 104 auf der anderen Seite fluiddurchlässig zu verbinden ist.

Senkrecht zu der Achse der beiden Durchgänge 119 befindet sich ein Ablasskanal 120, die den Ventilsitz 116 mit dem Inneren des nach außen geschlossenen Auffangbehälters 109 verbindet. Der Ventilsitz 116 ist einteilig aus Kunststoff mit dem Rohr 103 ausgeführt. In der geöffneten Position beziehungsweise Stellung des Dreiwege-Ventils 102 (Figur 12 unten, Figur 13 oben) ist der große durchgehende Kanal mit den beiden Durchgängen 119 verbunden und der kleine senkrechte Kanal im Dreiwege-Ventil 102 durch den Ventilsitz 116 geschlossen. Der Knochenzementteig aus der Kartusche kann dann aus dem Rohr 103 durch das Dreiwege-Ventil 102 und den Einsatz 118 in den Schlauch 104 fließen. In der geschlossenen Position beziehungsweise Stellung des Dreiwege-Ventils 102 (Figur 12 oben und Figur 13 unten) ist eine Seite des großen durchgehenden Kanals mit dem Ablasskanal 120 zum Innenraum des Auffangbehälters 109 verbunden und der kleinere senkrechte Kanal mit dem Durchgang 119 zum Schlauch 104 verbunden, während der andere Durchgang 119 zum Rohr 103 durch das Dreiwege-Ventil 102 verschlossen ist. Der Knochenzementteig kann so aus dem Schlauch 104 und gegebenenfalls aus einem an einem Luer-System-Adapter (nicht gezeigt), der am Schlauch 104 angeschlossen ist, und/oder einen angeschlossenen Trokar (nicht gezeigt) in den Auffangbehälter 109 fließen. Der Druck hierfür resultiert von einer elastischen Verformung des Schlauchs 104 und gegebenenfalls des Trokars, die sich beim Auspressen beziehungsweise beim Durchpressen des Knochenzementteigs aufgebaut hat.

Das Dreiwege-Ventil 102 kann über ein Bedienelement (nicht gezeigt), wie beispielsweise ein Knebelgriff (siehe das vorherige Ausführungsbeispiel), im Ventilsitz 116 manuell gedreht werden. Das außen zylindrische Dreiwege-Ventil 102 ist durch eine zylindrische Bohrung im Ventilsitz 116 geführt und auf der dem Bedienelement (nicht gezeigt) gegenüberliegenden Seite mit einem Stopfen (in den Figuren 12 und 13 nicht zu sehen aber analog dem vorherigen Ausführungsbeispiel ausgeführt) verbunden und so gegen Herausfallen oder versehentliches Herausziehen aus dem Ventilsitz 116 gesichert.

Durch den erfindungsgemäßen Aufbau gelingt es, dass der Strom des Knochenzementteigs durch Drehen und damit Schließen des Dreiwege-Ventils 102 schnell unterbrochen wird, ohne dass große Mengen des Knochenzementteigs durch eine Applikationsöffnung (nicht gezeigt) in den der Schlauch 104 oder der Trokar mündet, nachströmen. Gleichzeitig werden ein Austreten des Knochenzementteigs und damit eine Kontamination der Umgebung oder des Anwenders mit Hilfe des Auffangbehälters 109 verhindert, der überschüssigen Knochenzementteig aufnimmt. Des Weiteren bleibt der Druck in der Rückseite des Knochenzement-Applikators, also zwischen dem Dreiwege-Ventil 102 und dem Austragskolben der Kartusche, erhalten, so dass nach einem erneuten Öffnen des Dreiwege-Ventils 102 schnell wieder der Fluss des Knochenzementteigs bereitgestellt werden kann, ohne dass der Druck an der Rückseite Vorrichtung neu aufgebaut werden muss.

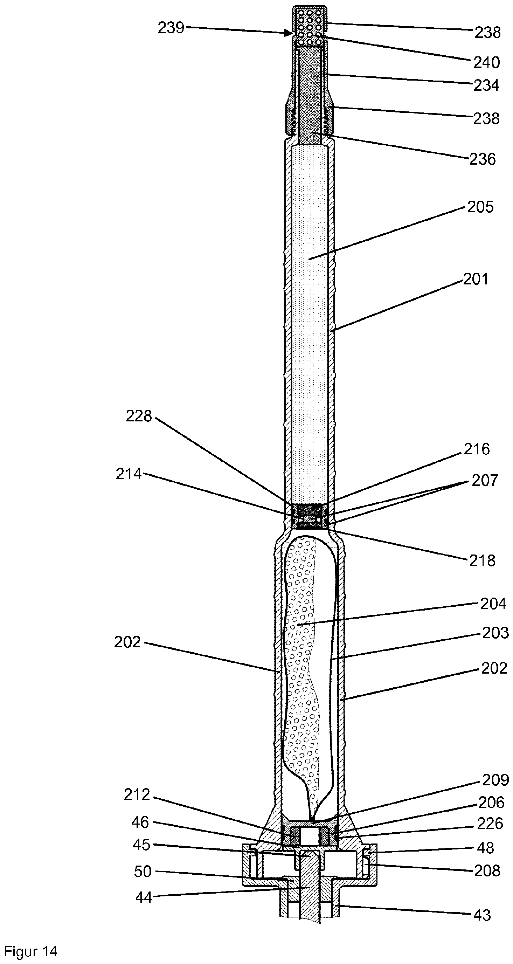

Die Figur 14 zeigt eine schematische Querschnittansicht einer dritten alternativen erfindungsgemäßen Vorrichtung zum Lagern und Mischen einer Monomerflüssigkeit 204 und eines Zementpulvers 205.

Der Aufbau der dritten alternativen erfindungsgemäßen Vorrichtung gleicht dort, wo es nicht anders beschrieben oder in Figur 14 zu sehen ist, dem ersten Ausführungsbeispiel nach den Figuren 1 bis 11.

Die Vorrichtung hat einen röhrenförmigen Behälter aus Kunststoff der als vorderen Teil (in Figur 14 oben) eine Kartusche 201 mit einem zylindrischen Innenraum aufweist und der eine Aufnahme 202 für einen Folienbeutel 203 aus Kunststoff, der mit einem Metall, wie Aluminium beschichtet sein kann, als hinteren Teil aufweist. Der röhrenförmige Behälter wird durch Spritzgießen hergestellt. Die Aufnahme 202 weist hierzu ebenfalls einen zylindrischen Innenraum auf, in den der Folienbeutel 203 eingesteckt ist. In dem Folienbeutel 203 befindet sich die Monomerflüssigkeit 204. In den Innenraum der Kartusche 201 ist ein Zementpulver 205 eingefüllt oder vorzugsweise eingepresst. Die Monomerflüssigkeit 204 und das Zementpulver 205 bilden die Ausgangskomponenten 204, 205 für einen PMMA-Knochenzement, der mit der Vorrichtung hergestellt werden kann. Mit Hilfe des Folienbeutels 203 kann die Monomerflüssigkeit 204 relativ lange in der Aufnahme 202 und dadurch in der Vorrichtung gelagert werden.

In der Aufnahme 202 ist ein im zylindrischen Innenraum der Aufnahme 202 in Längsrichtung beweglicher Förderkolben 206 aus Kunststoff angeordnet. Der Förderkolben 206 ist im Bereich der Rückseite der Aufnahme 202 angeordnet. Der Folienbeutel 203 kann mit dem Förderkolben 206 in der Aufnahme 202 zusammengedrückt und dabei zerquetscht oder aufgerissen werden, indem der Förderkolben 206 in Richtung der Vorderseite, also in Richtung der Kartusche 201 gedrückt wird. Der Folienbeutel 203 kann dazu an der Vorderseite des Förderkolbens 206 befestigt sein. Der Förderkolben 206 weist an der Vorderseite Abstreifer auf, mit denen Fetzen oder Reste des Folienbeutels 203 von der Innenwand der Aufnahme 202 abgestreift werden können.

In dem Innenraum der Kartusche 201 ist in deren Rückseite (in Figur 14 Richtung unten) ein Austragskolben 207 aus Kunststoff angeordnet. An der Rückseite der Aufnahme 202 ist ein Befestigungsmittel 208 vorgesehen, mit dem die Aufnahme 202 an einer Auspressvorrichtung (in Figur 14 nicht dargestellt) verbunden werden kann. Der röhrenförmige Behälter kann also über die Aufnahme 202 an der Auspressvorrichtung befestigt werden. Das Befestigungsmittel 208 ist vorzugsweise zur Bildung eines Bajonettverschlusses geeignet und vorgesehen. Dadurch kann der Förderkolben 206, der von der Rückseite der Aufnahme 202 aus frei zugänglich ist, mit der Auspressvorrichtung in Richtung der Vorderseite vorgetrieben werden.

Der Förderkolben 206 ist wie ein kurzes Rohr geformt, das an der seiner Vorderseite, die dem Folienbeutel 203 zugewandt ist, mit einer ebenen Wandung abgeschlossen ist. Die Wandung weist in der Mitte eine Sollbruchstelle 209 auf, die von der Rückseite des Förderkolbens 206 aus durch eine Vertiefung von hinten zugänglich ist. Die Stabilisierung und Abdichtung des restlichen Förderkolbens 206 wird mit einer röhrenförmigen Hülse 212 erreicht, die ebenfalls aus Kunststoff gefertigt ist und vorzugsweise als Gummimanschette 212 ausgeführt ist. Im Bereich der Sollbruchstelle 209 weist der Förderkolben 206 also eine verminderte Materialstärke auf. Dort kann der Folienbeutel 203 an dem Förderkolben 206 befestigt sein.

Die Kartusche 201 und die Aufnahme 202 sind einteilig als gemeinsames Kunststoffteil ausgeführt. Die Aufnahme 202 und die Kartusche 201 sind für die Monomerflüssigkeit 204 flüssigkeitsdurchlässig miteinander über eine Durchführung 214 im Austragskolben 207 verbunden. Die Durchführung 214 durch den Austragskolben 207 mündet durch einen für das Zementpulver 205 undurchlässigen aber für die Monomerflüssigkeit 204 durchlässigen Porenfilter 216 in den Innenraum der Kartusche 201.

In der Verbindung zur Durchführung 214 ist in dem röhrenförmigen Behälter ein Filter 218 angeordnet, mit dem die Fetzen und Bruchstücke des Folienbeutels 203 zurückgehalten werden können. Statt dem Filter 218 oder zusätzlich zum Filter 218 kann auch ein Sieb vorgesehen sein. Der Filter 218 ist in dem Austragskolben 207 angeordnet.

Mehrere Belüftungsöffnungen (in Figur 14 nicht zu sehen) sind in der Wandung der Aufnahme 202 vorgesehen, durch die der Innenraum der Aufnahme 202 mit Hilfe eines sterilisierenden Gases wie Ethylenoxid sterilisiert werden kann. Die Belüftungsöffnungen sind analog der Ausführung nach den Figuren 1 bis 11 unmittelbar benachbart zum Förderkolben 206 angeordnet, so dass der Förderkolben 206 die Belüftungsöffnungen unmittelbar verschließt, wenn er in Richtung der Kartusche 201 vorgetrieben wird. Dadurch wird verhindert, dass Monomerflüssigkeit 204 durch die Belüftungsöffnungen austreten kann, wenn der Folienbeutel 203 in der Aufnahme 202 geöffnet wurde.

Der zylindrische Förderkolben 206 hat einen zur Zylindergeometrie des Innenraums der Aufnahme 202 passenden Außenumfang und ist über zwei umlaufende Dichtungen 226 gegen die Innenwand der Aufnahme 202 flüssigkeitsdicht abgedichtet. Ebenso ist der Austragskolben 207 über zwei umlaufende Dichtungen 228 gegen die Innenwand der Kartusche 201 flüssigkeitsdicht abgedichtet. Diese Dichtungen 226, 228 dienen dazu, einen Austritt von Monomerflüssigkeit 204 oder von Knochenzement zu verhindern, um eine Kontamination der Umgebung (des OP-Saals und des Anwenders) zu verhindern. Die Dichtungen 226, 228 können hierzu aus Gummi bestehen.

Die Vorderseite der Kartusche 201 mündet in ein Austragsrohr 234, das ein Außengewinde aufweist. Im Inneren der Austragsrohrs 234 ist ein Porenfilter 236 angeordnet, der für das Zementpulver 205 undurchlässig aber für Gase durchlässig ist. An dem Außengewinde des Austragsrohrs 234 ist eine Kappe 238 befestigt, wobei der vordere Teil der Kappe 238 mit einem Styropor oder Schaumstoff 240 gefüllt ist. Die Kappe 238 kann von dem Austragsrohr 234 abgeschraubt werden kann. Die Kappe 238 weist seitliche Öffnungen 239 auf. Durch diesen Aufbau kann das Innere der Kartusche 201 und das Zementpulver 205 mit Hilfe von Ethylenoxid sterilisiert werden, da die Öffnungen 239 in der Kappe 238, das Styropor oder der Schaumstoff 240, der Porenfilter 236 und die Zwischenräume zwischen den Pulverpartikeln des Zementpulvers 205 luftdurchlässig sind. Gleichzeitig kann Luft aus der Aufnahme 2 durch das Zementpulver 205, den Porenfilter 236, das Styropor oder der Schaumstoff 240 und die Öffnungen 239 in der Kappe 238 herausgepresst werden, wenn der Förderkolben 206 in Richtung der Aufnahme 201 gepresst wird.

Das Zementpulver 205 ist in der Kartusche 201 eingeschlossen, da alle Öffnungen 239 und Durchgänge 214 mit Hilfe von den Porenfiltern 216, 236 für das Zementpulver 205 undurchlässig abgeschlossen sind. Der Inhalt der Kartusche 201 kann dabei durch Evakuieren und Spülen mit Ethylenoxid sterilisiert werden. Dadurch ist die Vorrichtung auch zum langfristigen Lagern des Zementpulvers 205 geeignet.

Im Folgenden wird der Ablauf eines erfindungsgemäßen Verfahrens anhand des dritten Ausführungsbeispiels diskutiert. Zu Beginn des Verfahrens liegt die Vorrichtung im Ausgangszustand vor, sowie sie auch in Figur 14 gezeigt ist. Der röhrenförmige Behälter 201, 202 der Vorrichtung wird in eine erfindungsgemäße Auspressvorrichtung 43 in Form einer Kartuschenpistole eingesetzt und mit dem Befestigungsmittel 208 an der Auspressvorrichtung 43 befestigt.

Die Auspressvorrichtung 43 umfasst eine linear vortreibbare Stange 44. Von der Auspressvorrichtung 43 ist nur der vordere Teil dargestellt in der sie sich von herkömmlichen Auspressvorrichtungen unterscheidet. Die Auspressvorrichtung 43 umfasst auch einen Griff und einen Kipphebel (in Figur 14 nicht zu sehen) zum manuellen Antreiben der Stange 44 der Auspressvorrichtung 43, wie bei herkömmlichen manuell angetriebenen Auspressvorrichtungen auch. Die Vorrichtung wird mit dem Befestigungsmittel 208 an der Auspressvorrichtung 43 befestigt. Die Stange 44 endet an ihrer Vorderseite in einer Spitze 45, die zunächst mit einer durchstechbaren Kappe 46 abgedeckt sein kann. Bevorzugt kann die Kappe 46 jedoch auch weggelassen werden, da der Folienbeutel 203 flexiblen genug ist und keine stabilen Scherben, wie eine Glasampulle ausbildet, so dass der Bewegung des Förderkolbens 206 keine derartig starke Kraft entgegenstehen wird, dass er bereits vor Erreichen der als Abstufung ausgeformten Anschlags zwischen der Aufnahme 202 und der Kartusche 201 den Förderkolben 206 an der Sollbruchstelle 209 durchstechen wird. Die Stange 44 drückt dann mit der Kappe 46 auf die Hülse 212 beziehungsweise Gummimanschette 212 des Förderkolbens 206 oder mit der Spitze 45 auf die Rückseite des Förderkolbens 206, wenn die Stange 44 von der Auspressvorrichtung 43 in die Aufnahme 202 hineingedrückt wird. Die Auspressvorrichtung 43 ist hierzu über ein Gegenbefestigungsmittel 48 an die Rückseite der Aufnahme 202 angeschlossen. Hierzu ist die Stange 44 gegen ein Lager 50 und darüber gegen das Gegenbefestigungsmittel 48 und damit gegen die Aufnahme 202 linear beweglich gelagert.

Die Auspressvorrichtung 43 wird bedient und dabei die Stange 44 und mit der Stange 44 der Förderkolben 6 in Richtung der Kartusche 1 vorgetrieben. Da der Folienbeutel 203 an der Vorderseite an dem Austragskolben 207 anliegt, verkleinert sich der Innenraum der Aufnahme 202 und der Folienbeutel 203 platzt auf oder zerreißt und die Monomerflüssigkeit 204 tritt aus dem Folienbeutel 203 in den Innenraum der Aufnahme 202 aus. Der Austragskolben 207 kann nicht von dem Folienbeutel 203 in Richtung des Porenfilters 236 geschoben werden, wenn das Zementpulver 205 trocken ist, also nicht von der Monomerflüssigkeit 204 benetzt ist, da das trockene Zementpulver 205 nicht fließfähig ist und eine Bewegung des Austragskolbens 207 blockiert. Überstehende Luft aus der Aufnahme 202 wird durch den Filter 218, die Durchführung 214, den Porenfilter 216, durch die Zwischenräume zwischen den Partikeln des Zementpulvers 205, durch den Porenfilter 236, durch den Schaumstoff 240 und aus den Öffnungen 239 in der Kappe 238 aus der Vorrichtung herausgedrückt.

Von dem Folienbeutel 203 bleiben schließlich Fetzen und Reste zurück, die von dem Filter 218 zurückgehalten werden und in dem röhrenförmigen Behälter verbleiben. Die Monomerflüssigkeit 204 wird durch den Filter 218, die Durchführung 214 und den Porenfilter 216 in das Zementpulver 205 gepresst und beginnt dort mit dem Zementpulver 205 zu reagieren, so dass sich aus dem Gemisch der Knochenzementteig bildet. Der Porenfilter 36 wird, sobald der Knochenzementteig entstanden ist, von dem auf den Knochenzementteig aufgrund des Drucks auf den Austragskolben 207 wirkenden Druck nach vorne getrieben und komprimiert den Schaumstoff 240. Wenn der Porenfilter 236 nun nach vorne rutscht, so wird er von außen für den Anwender durch die Öffnung 239 in der Kappe 238 sichtbar. Diese Situation ist analog in Figur 4 gezeigt. Hierzu hat der Porenfilter 236 vorzugsweise eine andere Farbe und Helligkeit als der Schaumstoff 240. Beispielsweise kann der Schaumstoff 240 weiß sein und der Porenfilter 236 rot.

In diesem Zustand wird die Kappe 238 mit dem Porenfilter 236 und dem Schaumstoff 240 abgeschraubt und stattdessen eine verlängerte Austragsöffnung auf das Austragsrohr 234 aufgeschraubt. Die verlängerte Austragsöffnung kann vorzugsweise ein Dreiwege-Ventil analog der ersten oder der zweiten Ausführung aufweisen. Ebenso kann an dem Austragsrohr 234 ein Schlauch und/oder ein Trokar angeschlossen werden. Die Stange 44 wird weiter angetrieben und durchsticht dabei mit der Spitze 45 den von dem Absatz zwischen der Kartusche 201 und der Aufnahme 202 blockierten Förderkolben 206 an der Sollbruchstelle 209 und gegebenenfalls zuvor die Kappe 46. Die Fetzen beziehungsweise Reste des Folienbeutels 203 werden dabei zur Seite gedrängt. Die Spitze 45 der Stange 44 trifft auf den Austragskolben 207.

Über den Schlauch und den Trokar oder über das Austragsrohr 234 kann der Knochenzementteig ausgetragen werden. Dazu wird der Austragskolben 207 mit der Stange 44 in Richtung des Austragsrohrs 234 vorgetrieben. Der Knochenzementteig aus dem Inneren der Kartusche 201 wird entweder durch das Austragsrohr 234 direkt oder bei geöffnetem Dreiwege-Ventil durch das Austragsrohr 234, durch das Dreiwege-Ventil, durch den Schlauch und den Trokar ausgetrieben und kann dort an den Wirbeln eines Patienten appliziert werden oder theoretisch zur weiteren Verarbeitung verwendet werden. Zur Unterbrechung des Flusses des Knochenzementteigs kann gegebenenfalls das Dreiwege-Ventil bedient werden.

Die Öffnungen 239 dienen auch als visuelle Marker anhand derer festgestellt werden kann, ob die Vorrichtung einsatzbereit ist. Wenn der Porenfilter 236 aufgrund des Drucks des Knochenzementteigs nach vorne gedrückt wird und dabei das Styropor oder Schaumstoff 240 in der Kappe 238 zusammenpresst, wird der Porenfilter 236 durch die Öffnungen 239 sichtbar. Dadurch kann der Anwender erkennen, dass der Knochenzementteig fertig gemischt in der Kartusche 201 vorliegt und damit einsatzbereit ist. Der Anwender kann zu diesem Zeitpunkt am Außengewinde des Austragsrohrs 234 einen geeigneten Aufbau, wie beispielsweise den Trokar mit dem Schlauch befestigen und den Förderkolben 206 an der Sollbruchstelle 209 mit der Spitze 45 der Stange 44 durchstoßen und so den Austragskolben 207 antreiben und dadurch den Knochenzementteig aus der Kartusche 201 austreiben.

### Bezugszeichenliste

- 1,201: Kartusche
- 2,202: Aufnahme
- 3: Ampulle
- 4, 204: Monomerflüssigkeit
- 5, 205: Zementpulver
- 6, 206: Förderkolben
- 7, 207: Austragskolben
- 8, 208: Befestigungsmittel / Bajonettverschluss
- 9, 209: Sollbruchstelle
- 10: Vertiefung
- 12, 212: Hülse / Gummimanschette
- 14, 214: Durchführung
- 16, 216: Porenfilter
- 18, 218: Filter
- 20: Belüftungsöffnung
- 26, 226: Dichtung
- 28, 228: Dichtung
- 34, 234: Austragsrohr
- 36, 236: Porenfilter
- 38, 238: Kappe
- 39, 239: Öffnung
- 40, 240: Schaumstoff
- 42: Flügel
- 43: Auspressvorrichtung / Kartuschenpistole
- 44: Stange
- 45: Spitze
- 46: Kappe
- 48: Gegenbefestigungsmittel / Bajonettverschluss
- 50: Lagerung
- 52: Splitter
- 54: Knochenzementteig / Gemisch
- 56: Dreiwege-Ventil
- 58: Knebelgriff
- 59: Ventilsitz / Rohr
- 60: Auffangbehälter
- 61: Durchgang
- 62, 78: Flügel
- 64: Schlauch
- 66: Trokar
- 68: Trokarspitze
- 72: Anschluss
- 74: Luer-System-Adapter
- 76: Schlauch
- 80: Einsatz
- 82: Hülse
- 84: Dichtung
- 86: Stopfen
- 88: Ablasskanal
- 102: Dreiwege-Ventil
- 103: Rohr
- 104: Schlauch
- 109: Auffangbehälter
- 112: Hülse
- 114: Statischer Mischer
- 116: Ventilsitz
- 118: Einsatz
- 119: Durchgang
- 120: Ablasskanal
- 203: Folienbeutel

## Patentansprüche

1. Vorrichtung zum Lagern einer Monomerflüssigkeit (4, 204) und eines Zementpulvers (5, 205) als Ausgangskomponenten eines Knochenzementteigs (54) sowie zum Mischen des Knochenzementteigs (54) aus den Ausgangskomponenten und zum Austragen des gemischten Knochenzementteigs (54), die Vorrichtung aufweisend einen röhrenförmigen Behälter, der an seiner Rückseite eine Aufnahme (2, 202) mit einem zylindrischen Innenraum bildet, in der ein Monomerflüssigkeitsbehälter (3, 203) angeordnet ist, wobei in dem Monomerflüssigkeitsbehälter (3, 203) die Monomerflüssigkeit (4, 204) enthalten ist, und der Behälter an seiner Vorderseite eine Kartusche (1, 201) mit einem zylindrischen Innenraum bildet, in der das Zementpulver (5, 205) enthalten ist, wobei
im Innenraum der Aufnahme (2, 202) ein in Längsrichtung der Aufnahme (2, 202) beweglicher Förderkolben (6, 206) angeordnet ist, der von einer Rückseite der Aufnahme (2, 202) aus zugänglich ist, wobei
im Innenraum der Kartusche (1, 201) ein in Längsrichtung im Inneren der Kartusche (1, 201) verschiebbarer Austragskolben (7, 207) zwischen dem Monomerflüssigkeitsbehälter (3, 203) und dem Zementpulver (5, 205) angeordnet ist, wobei
der Innenraum der Aufnahme (2, 202) und der Innenraum der Kartusche (1, 201) über eine für die Monomerflüssigkeit (4, 204) und für Gase durchlässige aber für das Zementpulver (5, 205) undurchlässige Verbindung (14, 214) miteinander verbunden sind, und wobei
der Förderkolben (6, 206) von der Rückseite aus mit einer Stange (44) durchstechbar ist, wenn eine Bewegung des Förderkolbens (6, 206) in Richtung der Vorderseite des Behälters blockiert ist, wobei der Austragskolben (7, 207) durch ein weiteres Vortreiben der Stange (44) durch den blockierten und durchstochenen Förderkolben (6, 206) hindurch in Richtung der Vorderseite der Kartusche (1, 201) vortreibbar ist.

2. Vorrichtung nach Anspruch 1, **dadurch gekennzeichnet, dass**
der röhrenförmige Behälter einteilig ist, wobei vorzugsweise die Aufnahme (2, 202) und die Kartusche (1, 201) als ein einteiliger thermoplastischer Kunststoffkörper aufgebaut sind, wobei der Behälter besonders bevorzugt mit einem Spitzgussverfahren hergestellt ist.

3. Vorrichtung nach einem der vorangehenden Ansprüche, **dadurch gekennzeichnet, dass**
die Querschnittsfläche des Innenraums der Kartusche (1, 201) kleiner ist als die Querschnittsfläche des Innenraums der Aufnahme (2, 202).

4. Vorrichtung nach einem der vorangehenden Ansprüche, **dadurch gekennzeichnet, dass**
an dem röhrenförmigen Behälter, insbesondere an der Rückseite der Aufnahme (2, 202), ein Befestigungsmittel (8, 208) zur Befestigung einer Auspressvorrichtung (43) angeordnet ist.

5. Vorrichtung nach einem der vorangehenden Ansprüche, **dadurch gekennzeichnet, dass**
im Inneren des Behälters ein Anschlag vorgesehen ist, der die Bewegung des Förderkolbens (6, 206) in Richtung der Vorderseite begrenzt, wobei der Anschlag die Bewegung vorzugsweise derart begrenzt, dass der Förderkolben (6, 206) nicht vollständig aus der Aufnahme (2, 202) herauspressbar ist, wobei besonders bevorzugt der Anschlag als Abstufung durch eine unterschiedliche Form oder unterschiedliche Querschnitte des zylindrischen Innenraums der Kartusche (1, 201) und des zylindrischen Innenraums der Aufnahme (2, 202) am Übergang zwischen der Aufnahme (2, 202) und der Kartusche (1, 201) gebildet ist.

6. Vorrichtung nach einem der vorangehenden Ansprüche, **dadurch gekennzeichnet, dass**
die Verbindung (14, 214) im Austragskolben (7, 207) angeordnet ist und/oder in der Wandung des röhrenförmigen Behälters angeordnet ist, wobei vorzugsweise die Verbindung (14, 214) im Austragskolben (7, 207) angeordnet ist und die Vorderseite des Austragskolbens (7, 207) mit der dem Monomerflüssigkeitsbehälter (3, 203) zugewandten Rückseite des Austragskolbens (7, 207) verbindet.

7. Vorrichtung nach einem der vorangehenden Ansprüche, **dadurch gekennzeichnet, dass**
an der Einmündung der Verbindung (14, 214) in den Innenraum der Kartusche (1, 201) ein für Gase und die Monomerflüssigkeit (4, 204) durchlässiger aber für das Zementpulver (5, 205) undurchlässiger Filter (16, 216), insbesondere Porenfilter (16, 216), angeordnet ist.

8. Vorrichtung nach einem der vorangehenden Ansprüche, **dadurch gekennzeichnet, dass**
das Zementpulver (5, 205) an der Vorderseite des Austragskolbens (7, 207) anliegt, insbesondere vollflächig anliegt, wobei vorzugsweise das Zementpulver (5, 205) in den Innenraum der Kartusche (1, 201) eingepresst ist.

9. Vorrichtung nach einem der vorangehenden Ansprüche, **dadurch gekennzeichnet, dass**
die Kartusche (1, 201) an der Vorderseite eine Austragsöffnung aufweist, die mit einem Verschluss (36, 236) verschlossen ist, wobei der Knochenzementteig (54) durch die Austragsöffnung aus der Kartusche (1, 201) auspressbar ist, wenn die Austragsöffnung geöffnet ist, wobei der Verschluss (36, 236) für Gase durchlässig und für das Zementpulver (5, 205) undurchlässig ist, wobei
an der Vorderseite der Kartusche (1, 201) ein Austragsrohr (34, 234) angeordnet und/oder ein flexibler Schlauch (64) mit einem Trokar (66) befestigt ist oder befestigbar ist, wobei der Knochenzementteig (54) durch das Austragsrohr (34, 234) und/oder den flexiblen Schlauch (64) und den Trokar (66) auspressbar ist, wobei vorzugsweise an dem Austragsrohr (34, 234) oder dem Schlauch (64) ein manuell verschließbares Ventilelement angeordnet ist, mit dem sich der Fluss des Knochenzementteigs (54) steuern lässt.

10. Vorrichtung nach einem der vorangehenden Ansprüche, **dadurch gekennzeichnet, dass**
der Förderkolben (6, 206) von der Rückseite aus mit der Stange (44) aufweisend eine Spitze (45) oder eine Kante durchstechbar ist, wenn eine weitere Bewegung des Förderkolbens (6, 206) in Richtung der Vorderseite des röhrenförmigen Behälters blockiert ist, und/oder
der Förderkolben (6, 206) von der Rückseite aus mit der Stange (44) mit einer Kraft von mindestens 1 kN durchstechbar ist, wenn eine weitere Bewegung des Förderkolbens (6, 206) in Richtung der Vorderseite des röhrenförmigen Behälters blockiert ist, und/oder
der Förderkolben (6, 206) im Bereich der Angriffsfläche der Stange (44) eine maximale Dicke von maximal 4 mm, bevorzugt von maximal 3 mm und ganz besonders bevorzugt von maximal 2 mm hat.

11. Vorrichtung nach einem der vorangehenden Ansprüche, **dadurch gekennzeichnet, dass**
der Querschnitt des Innenraums der Kartusche (1, 201) maximal 4 cm² beträgt, bevorzugt maximal 2,5 cm² beträgt, besonders bevorzugt maximal 1,2 cm² beträgt, oder
der Monomerflüssigkeitsbehälter (3, 203) eine Glasampulle (3), eine Plastikampulle (3), ein Kunststofffolienbeutel (203) oder ein Aluminium-Kunststoff-Verbund-Beutel (203) ist.

12. Vorrichtung nach einem der vorangehenden Ansprüche, **dadurch gekennzeichnet, dass**
das Volumen der Monomerflüssigkeit (4, 204) im Monomerflüssigkeitsbehälter (3, 203) mindestens so groß ist wie das Volumen der mit Luft gefüllten Zwischenräume zwischen den Zementpulverpartikeln in der Kartusche (1, 201), bevorzugt mindestens so groß ist wie das Volumen der Flüssigkeitsleitungen zwischen dem Innenraum der Kartusche (1, 201) und dem Innenraum der Aufnahme (2, 202) plus das Volumen der mit Luft gefüllten Zwischenräume zwischen den Zementpulverpartikeln in der Kartusche (1, 201).

13. Vorrichtung nach einem der vorangehenden Ansprüche, **dadurch gekennzeichnet, dass**
in der Wandung der Aufnahme (2, 202) zumindest eine Belüftungsöffnung (20) angeordnet ist, die den Innenraum der Aufnahme (2, 202), in dem der Monomerflüssigkeitsbehälter (3, 203) angeordnet ist, mit der Umgebung verbindet, wobei bevorzugt die zumindest eine Belüftungsöffnung (20) derart dicht im Bereich des Förderkolbens (6, 206) angeordnet ist, dass sie durch eine Bewegung des Förderkolbens (6, 206) in Richtung der Vorderseite der Aufnahme (2, 202) verschlossen wird, bevor der Monomerflüssigkeitsbehälter (3, 203) durch die Bewegung des Förderkolbens (6, 206) geöffnet ist.

14. Vorrichtung nach einem der vorangehenden Ansprüche, **dadurch gekennzeichnet, dass**
an der Vorderseite der Aufnahme (2, 202) in der Verbindung (14, 214) zur Kartusche (1, 201) ein Sieb oder eine für Gase und Flüssigkeiten durchlässige poröse Scheibe (18, 218) vorgesehen ist, oder
ein von außen bedienbares Dreiwege-Ventil (56, 102) in der Flussrichtung des Knochenzementteigs (54) in einer Leitung hinter der Kartusche (1, 201) angeordnet ist, ein Auffangbehälter (60, 109) zum Aufnehmen von Knochenzementteig (54) an dem Dreiwege-Ventil (56, 102) angeordnet ist, wobei Leitung in eine Applikationsöffnung mündet, die an dem von der Kartusche (1, 201) abgewandten Ende der Leitung angeordnet ist, wobei das Dreiwege-Ventil (56, 102) derart aufgebaut ist, dass es in einer ersten Stellung eine Fluidverbindung zwischen der Applikationsöffnung und der Kartusche (1, 201) bereitstellt und einen Ablasskanal (88, 120) zum Auffangbehälter (60, 109) verschließt und
in einer zweiten Stellung eine Fluidverbindung zwischen der Applikationsöffnung und dem Auffangbehälter (60, 109) bereitstellt und einen Durchgang zur Kartusche (1, 201) verschließt.

15. Vorrichtung nach einem der vorangehenden Ansprüche, **dadurch gekennzeichnet, dass**
der Monomerflüssigkeitsbehälter (3, 203) im Inneren der Aufnahme (2, 202) durch eine Bewegung des Förderkolbens (6, 206) in Richtung der Vorderseite der Aufnahme (2, 202) zu öffnen ist, bevorzugt aufzubrechen oder aufzureißen ist, oder
der Förderkolben (6, 206) an der rückseitigen Stirnseite eine Aufnahme (2, 202) oder eine Vertiefung (10), insbesondere eine zylindrische Vertiefung (10), für die Stange (44) der Auspressvorrichtung (43) aufweist, in der bevorzugt eine Gummimanschette (12, 212) als Dichtungselement angeordnet ist oder eine von der Stange (44) durchstechbare, plastisch verformbare Kunststoff- oder Metallscheibe (9, 209) als Dichtungselement eingesetzt ist.

16. Verfahren zur Herstellung eines Knochenzementteigs (54), insbesondere eines pastenförmigen Polymethylmethacrylat-Knochenzementteigs (54), wobei der Knochenzementteig (54) aus einem Zementpulver (5, 205) und einer Monomerflüssigkeit (4, 204) mit einer Vorrichtung hergestellt wird, wobei die Vorrichtung röhrenförmigen Behälter aufweist, der an seiner Rückseite eine Aufnahme (2, 202) mit einem zylindrischen Innenraum bildet, in der ein Monomerflüssigkeitsbehälter (3, 203) mit der Monomerflüssigkeit (4, 204) darin angeordnet ist, und der Behälter an seiner Vorderseite eine Kartusche (1, 201) mit einem zylindrischen Innenraum bildet, in der das Zementpulver (5, 205) enthalten ist, **gekennzeichnet durch** die folgenden nacheinander ablaufenden Schritte:
a) Einsetzen der Vorrichtung in eine Auspressvorrichtung (43), die Auspressvorrichtung (43) aufweisend eine axial vortreibbare Stange (44),
b) ein Förderkolben (6, 206), der innerhalb der Aufnahme (2, 202) an deren Rückseite beweglich gelagert ist, wird mit der Stange (44) in Richtung der Kartusche (1, 201) vorgetrieben, wobei **durch** die Bewegung des Förderkolbens (6, 206) der Monomerflüssigkeitsbehälter (3, 203) geöffnet wird und die Monomerflüssigkeit (4, 204) aus der Aufnahme (2, 202) in die Kartusche (1, 201) gepresst wird, wobei im Innenraum der Kartusche (1, 201) sich das Zementpulver (5, 205) mit der Monomerflüssigkeit (4, 204) mischt,
c) die Bewegung des Förderkolbens (6, 206) in Richtung der Vorderseite des Behälters wird blockiert,
d) die Stange (44) sticht **durch** den Förderkolben (6, 206) und trifft nach dem Durchstechen des Förderkolbens (6, 206) auf einen Austragskolben (7, 207), der innerhalb der Kartusche (1, 201) beweglich gelagert ist,
e) der Austragskolben (7, 207) wird mit der Stange (44) in Richtung der Vorderseite der Kartusche (1, 201) vorgetrieben, während die Stange (44) **durch** den blockierten und durchstochenen Förderkolben (6, 206) hindurch läuft, wobei **durch** die Bewegung des Austragskolbens (7, 207) die Mischung aus dem Zementpulver (5, 205) und der Monomerflüssigkeit (4, 204) aus der Kartusche (1, 201) als Knochenzementteig (54) ausgetrieben wird.

17. Verfahren nach Anspruch 16, **dadurch gekennzeichnet, dass**
das Verfahren mit einer Vorrichtung nach einem der Ansprüche 1 bis 15 umgesetzt wird.

18. Verfahren nach Anspruch 16 oder 17, **dadurch gekennzeichnet, dass** das Verfahren mit einer Auspressvorrichtung (43) nach einem der Ansprüche 13 bis 15 umgesetzt wird.

19. Verfahren nach einem der Ansprüche 16 bis 18, **dadurch gekennzeichnet, dass**
die Stange (44) an der Vorderseite eine harte Spitze (45) oder Kante zum Durchstechen des blockierten Förderkolbens (6, 206) der Vorrichtung aufweist, wobei in Schritt a) auf der Spitze (45) oder Kante eine abnehmbare Kappe (46) mit einer ebenen Vorderseite angeordnet ist und die Kappe (46) nach Schritt c) von der Spitze (45) oder Kante entfernt wird und die Stange (44) mit der Spitze (45) oder der Kante in den Förderkolben (6, 206) getrieben wird, wobei vorzugsweise zuvor die Vorrichtung aus der Auspressvorrichtung (43) entnommen wird und die Vorrichtung nach Entfernen der Kappe (46) von der Spitze (45) oder Kante wieder in die Auspressvorrichtung (43) eingesetzt wird, oder dass
vor Schritt c) oder in Schritt c) durch den auf die Mischung des Zementpulvers (5, 205) mit der Monomerflüssigkeit (4, 204) wirkenden Druck ein Verschluss (36, 236), insbesondere ein Porenfilter (36, 236), in einer Austragsöffnung an der Vorderseite der Kartusche (1, 201) bewegt oder hervorgedrückt wird, wobei hierauf vorzugsweise der Verschluss (36, 236)aus der Austragsöffnung entfernt wird und ein verlängertes Austragsrohr oder ein Schlauch (64) mit einem Trokar (66) an der Vorderseite der Kartusche (1, 201) befestigt wird, und/oder die Kappe (46) von der Spitze (45) oder der Kante der Stange (44) entfernt wird.

20. Verfahren nach einem der Ansprüche 16 bis 19, **dadurch gekennzeichnet, dass**
der zerbrochene oder aufgeschlitzte oder aufgeplatzte Monomerflüssigkeitsbehälter (3, 203) in Schritt b) zusammengeschoben wird und gleichzeitig Gas aus der Aufnahme (2, 202) durch eine Verbindung (14, 214) in die Kartusche (1, 201) gedrückt wird und durch das Zementpulver (5, 205) in der Kartusche (1, 201) nach außen gedrückt wird und wobei in Schritt d) die durch den Förderkolben (6, 206) getriebene Stange (44) die Bruchstücke (52) des Monomerflüssigkeitsbehälters (3, 203) verdrängt, und/oder in Schritt d) unmittelbar vor der Applikation des Knochenzementteigs (54) an der Vorderseite der Kartusche (1, 201) an einer Austragsöffnung ein Trokar (66) mit einem Schlauch (64) mit der Kartusche (1, 201) verbunden wird und der Knochenzementteig (54) anschließend durch den Schlauch (64) und den Trokar (66) und gegebenenfalls ein Austragsrohr (34, 234) infolge der Vorwärtsbewegung des Austragskolbens (7, 207) durch weitere Betätigung der Auspressvorrichtung (43) ausgepresst wird.

## Claims

1. Device for storage of a monomer liquid (4, 204) and a cement powder (5, 205) as starting components of a bone cement dough (54) and for mixing of the bone cement dough (54) from the starting components, and for dispensing the mixed bone cement dough (54), the device comprising
a tube-shaped container that forms, on its rear side, a receptacle (2, 202) with a cylindrical internal space, in which a monomer liquid container (3, 203) is arranged, whereby the monomer liquid container (3, 203) contains the monomer liquid (4, 204), and the container forms, on its front side, a cartridge (1, 201) with a cylindrical internal space that contains the cement powder (5, 205),
a feed plunger (6, 206) which is movable in a longitudinal direction of the receptacle (2, 202) and which is accessible from a rear side of the receptacle (2, 202), whereby the feed plunger (6, 206) is arranged in the internal space of the receptacle (2, 202),
a dispensing plunger (7, 207) which is movable in a longitudinal direction in the internal space of the cartridge (1, 201), whereby the dispensing plunger (7, 207) is arranged between the monomer liquid container (3, 203) and the cement powder (5, 205) in the internal space of the cartridge (1, 201), whereby
the internal space of the receptacle (2, 202) and the internal space of the cartridge (1, 201) are connected to each other through a connection (14, 214) that is permeable to the monomer liquid (4, 204) and gases, but is impermeable to the cement powder (5, 205), and whereby
the feed plunger (6, 206) is puncturable, from the rear side, by a rod (44), when a motion of the feed plunger (6, 206) in the direction of the front side of the container is blocked, whereby the dispensing plunger (7, 207) is propellable by propelling the rod (44) further through the blocked and punctured feed plunger (6, 206) in the direction of a front side of the cartridge (1, 201).

2. Device according to claim 1, **characterised in that**
the tube-shaped container has a one-part design, whereby the receptacle (2, 202) and the cartridge (1, 201) are preferably designed as a one-part thermoplastic resin body, whereby the container is particularly preferably manufactured using an injection moulding process.

3. Device according to any one of the preceding claims, **characterised in that**
the cross-sectional surface area of the internal space of the cartridge (1,201) is smaller than the cross-sectional surface area of the internal space of the receptacle (2, 202).

4. Device according to any one of the preceding claims, **characterised in that**
a securing means (8, 208) for securing an extrusion device (43) is arranged on the tube-shaped container, in particular on the rear side of the receptacle (2, 202).

5. Device according to any one of the preceding claims, **characterised in that**
a limit stop is provided on the inside of the container that limits the motion of the feed plunger (6, 206) in the direction of the front side, whereby the limit stop preferably limits the motion appropriately such that the feed plunger (6, 26) cannot be fully pressed out of the receptacle (2, 202), whereby the limit stop is particularly preferably provided as a step arising from different shape or different cross-sections of the cylindrical internal space of the cartridge (1, 201) and the cylindrical internal space of the receptacle (2, 202) at the transition between the receptacle (2, 202) and the cartridge (1, 201).

6. Device according to any one of the preceding claims, **characterised in that**
the connection (14, 214) is arranged in the dispensing plunger (7, 207) and/or is arranged in the wall of the tube-shaped container, whereby the connection (14, 214) is preferably arranged in the dispensing plunger (7, 207) and connects a front side of the dispensing plunger (7, 207) to a rear side of the dispensing plunger (7, 207) that faces the monomer liquid container (3, 203).

7. Device according to any one of the preceding claims, **characterised in that**
a filter (16, 216), in particular a pore filter (16, 216), that is permeable to gases and the monomer liquid (4, 204), but is impermeable to the cement powder (5, 205), is arranged at the merging site of the connection (14, 214) into the internal space of the cartridge (1,201).

8. Device according to any one of the preceding claims, **characterised in that**
the cement powder (5, 205) touches against a front side of the dispensing plunger (7, 207), in particular across the entire surface, whereby the cement powder (5, 205) is preferably pressed into the internal space of the cartridge (1, 201).

9. Device according to any one of the preceding claims, **characterised in that**
the cartridge (1, 201) comprises, on the front side, a dispensing opening that is closed by a closure (36, 236), whereby the bone cement dough (54) is extrudable from the cartridge (1, 201) through the dispensing opening, if the dispensing opening is open, whereby the closure (36, 236) is permeable to gases and impermeable to the cement powder (5, 205), whereby
a dispensing tube (34, 234) and/or a flexible hose (64) with a trocar (66) is secured or securable to the front side of the cartridge (1, 201), whereby the bone cement dough (54) is extrudable through the dispensing tube (34, 234) and/or the flexible hose (64) and the trocar (66), whereby a manually closable valve element, which is usable to control the flow of bone cement dough (54), is arranged on the dispensing tube (34, 234) or hose (64).

10. Device according to any one of the preceding claims, **characterised in that**
the feed plunger (6, 206) is puncturable from a rear side by the rod (44) comprising a tip (45) or an edge, if the feed plunger (6, 206) is blocked from moving further in the direction of the front side of the tube-shaped container, and/or
the feed plunger (6, 206) is puncturable from a rear side by the rod (44) with a force of at least 1 kN, if the feed plunger (6, 206) is blocked from moving further in the direction of the front side of the tube-shaped container, and/or
the feed plunger (6, 206) has a maximum thickness in the area of the contact surface of the rod of at most 4 mm, preferably of at most 3 mm, and particularly preferably of at most 2 mm.

11. Device according to any one of the preceding claims, **characterised in that**
the cross-section of the internal space of the cartridge (1, 201) is at most 4 cm², preferably at most 2.5 cm², particularly preferably at most 1.2 cm², or
the monomer liquid container (3, 203) is a glass ampoule (3), a plastic ampoule (3), a plastic film bag (203) or an aluminium-plastic compound bag (203).

12. Device according to any one of the preceding claims, **characterised in that**
the volume of the monomer liquid (4, 204) in the monomer liquid container (3, 203) is at least as large as the volume of the air-filled intervening spaces between the cement powder particles in the cartridge (1, 201), preferably is at least as large as the volume of the liquid conduits between the internal space of the cartridge (1, 201) and the internal space of the receptacle (2, 202) plus the volume of the air-filled intervening spaces between the cement powder particles in the cartridge (1, 201).

13. Device according to any one of the preceding claims, **characterised in that**
at least one ventilation opening (20) is arranged in the wall of the receptacle (2, 202), whereby the ventilation opening (20) connects the internal space of the receptacle (2, 202), in which the monomer liquid container (3, 203) is arranged, to the surroundings, whereby the at least one ventilation opening (20) is preferably arranged sufficiently close to the feed plunger (6, 206) such that it is closed by a motion of the feed plunger (6, 26) in the direction of a front side of the receptacle (2, 202) before the monomer liquid container (3, 203) is opened through the motion of the feed plunger (6, 206).

14. Device according to any one of the preceding claims, **characterised in that**
a screen or a porous disk (18, 218) that is permeable to gases and liquids is provided on a front side of the receptacle (2, 202) in the connection (14, 214) to the cartridge (1, 201), or
a three-way valve (56, 102) that is operable from outside is arranged in the flow direction of the bone cement dough (54) in a conduit downstream from the cartridge (1, 201),
a collecting container (60, 109) for reception of bone cement dough (54) is arranged on the three-way valve (56, 102), whereby the conduit merges into an application opening that is arranged on the end of the conduit that faces away from the cartridge (1, 201), whereby the three-way valve (56, 102) is designed appropriately such that it, being in a first position, provides a fluid connection between the application opening and the cartridge (1, 201) and closes a discharge channel (88, 120) toward the collecting container (60, 109) and,
being in a second position, provides a fluid connection between the application opening and the collecting container (60, 109) and closes a passage to the cartridge (1, 201).

15. Device according to any one of the preceding claims, **characterised in that**
the monomer liquid container (3, 203) is openable on the inside of the receptacle (2, 202) through a motion of the feed plunger (6, 206) in the direction of a front side of the receptacle (2, 202), preferably is break-openable or tear-openable, or
the feed plunger (6, 206) comprises, on the rear-side face, a receptacle (2, 202) or a recess (10), in particular a cylindrical recess (10), for the rod (44) of the extrusion device (43), in which a rubber cuff (12, 212) is preferably arranged as sealing element or in which a plastic or metal disk (9, 209) as sealing element that is plastically deformable and is puncturable by the rod (44).

16. Method for the production of a bone cement dough (54), in particular of a pasty polymethylmethacrylate bone cement dough (54), whereby the bone cement dough (54) is produced from a cement powder (5, 205) and a monomer liquid (4, 204) through the use of a device, whereby the device comprises tube-shaped container that forms, on its rear side, a receptacle (2, 202) with a cylindrical internal space, in which a monomer liquid container (3, 23) with the monomer liquid (4, 204) in it is arranged, and the container forms, on its front side, a cartridge 1, 201) with a cylindrical internal space that contains the cement powder (5, 205), **characterised by** the following steps proceeding in the order given:
a) inserting the device in an extrusion device (43), whereby the extrusion device (43) comprises a rod (44) that is propellable in axial direction;
b) a feed plunger (6, 206), supported such as to be mobile in the receptacle (2, 202) on a rear side thereof, is propelled in the direction of the cartridge by the rod (44), whereby the motion of the feed plunger (6, 206) opens the monomer liquid container (3, 203) and presses the monomer liquid (4, 204) from the receptacle (2, 202) into the cartridge (1, 201), whereby the cement powder (5, 205) mixes with the monomer liquid (4, 204) in the internal space of the cartridge (1, 201);
c) the motion of the feed plunger (6, 206) in the direction of the front side of the container is blocked;
d) the rod (44) punctures the feed plunger (6, 26) and after puncturing the feed plunger (6, 26) hits on a dispensing plunger (7, 207) that is supported in the cartridge (1, 201) such as to be mobile;
e) the dispensing plunger (7, 207) is propelled in the direction of the front side of the cartridge (1, 201) by the rod (44), while the rod (44) runs through the blocked and punctured feed plunger (6, 206), whereby the mixture of cement powder (5, 205) and monomer liquid (4, 204) is expelled from the cartridge (1, 201) as bone cement dough (54) due to the motion of the dispensing plunger (7, 207).

17. Method according to claim 16, **characterised in that**
the method is implemented using a device according to any one of the claims 1 to 15.

18. Method according to claim 16 or 17, **characterised in that**
the method is implemented using an extrusion device (43) according to any one of the claims 13 to 15.

19. Method according to any one of the claims 16 to 18, **characterised in that**
the rod (44) comprises, on the front side, a hard tip (45) or edge for puncturing the blocked feed plunger (6, 206) of the device, whereby, in step a), a removable cap (46) with a level front side is arranged on the tip (45) or edge, and the cap (46) is removed from the tip (45) or edge after step c), and the rod (44) is driven into the feed plunger (6, 26) by the tip (45) or edge, whereby the device is preferably being removed from the extrusion device (43) earlier and the device is re-inserted into the extrusion device (43) after removing the cap (46) from the tip (45) or edge, or that a closure (36, 236), in particular a pore filter (36, 236), is moved in or pushed out of a dispensing opening on the front side of the cartridge (1, 201) before step c) or in step c) by the pressure acting on the mixture of cement powder (5, 205) and monomer liquid (4, 204), whereby the closure (36, 236) is then preferably removed from the dispensing opening and an extended dispensing tube or a hose (64) with a trocar (66) is secured to the front side of the cartridge (1, 201) and/or the cap (46) is removed from the tip (45) or edge of the rod (44).

20. Method according to any one of the claims 16 to 19, **characterised in that**
the crushed or slit-open or burst-open monomer liquid container (3, 23) is pushed together in step b) and simultaneously gas is pushed from the receptacle (2, 202) through a connection (14, 214) into the cartridge (1, 201) and is pushed through the cement powder (5, 205) in the cartridge (1, 201) to the outside, and whereby, in step d), the rod (44) driven through the feed plunger (6, 206) displaces the fragments (52) of the monomer liquid container (3, 203), and/or
in step d) right before the application of the bone cement dough (54), a trocar (66) with a hose (64) is connected to the cartridge (1, 201) on the front side of the cartridge (1, 201) on a dispensing opening and the bone cement dough (54) subsequently is extruded through the hose (64) and the trocar (66) and, if applicable, a dispensing tube (34, 234) due to the forward motion of the dispensing plunger (7, 207) by further actuation of the extrusion device (43).

## Revendications

1. Dispositif de stockage d'un liquide monomère (4, 204) et d'une poudre de ciment (5, 205) servant de composants de départ d'une pâte de ciment osseux (54), ainsi que pour le mélange de la pâte de ciment osseux (54) à partir des composants de départ et pour l'évacuation de la pâte de ciment osseux (54) mélangée, le dispositif présentant un récipient en forme de tube qui forme un réceptacle (2, 202) avec un espace intérieur cylindrique au niveau de sa face arrière, dans lequel est disposé un récipient de liquide monomère (3, 203), où le liquide monomère (4, 204) est contenu dans le récipient de liquide monomère (3, 203), et le récipient forme au niveau de sa face avant une cartouche (1, 201) avec un espace intérieur cylindrique, dans lequel la poudre de ciment (5, 205) est contenue, où
un piston de transport (6, 206), qui est accessible par une face arrière du réceptacle (2, 202), mobile dans la direction longitudinale du réceptacle (2, 202), est disposé dans l'espace intérieur du réceptacle (2, 202), où un piston de sortie (7, 207) pouvant coulisser dans la direction longitudinale à l'intérieur de la cartouche (1, 201) est disposé dans l'espace intérieur de la cartouche (1, 201) entre le récipient de liquide monomère (3, 203) et la poudre de ciment (5, 205),
où
l'espace intérieur du réceptacle (2, 202) et l'espace intérieur de la cartouche (1, 201) sont reliés l'un à l'autre par le biais d'une liaison (14, 214) perméable au liquide monomère (4, 204) et aux gaz, mais imperméable pour la poudre de ciment (5, 205), et où
le piston de transport (6, 206) peut être transpercé à partir de la face arrière avec une tige (44) lorsqu'un mouvement du piston de transport (6, 206) est bloqué en direction de la face avant du récipient, où le piston de sortie (7, 207) peut être actionné vers l'avant par un nouveau mouvement vers l'avant de la tige (44) à travers le piston de transport (6, 206) bloqué et transpercé en direction de la face avant de la cartouche (1, 201).

2. Dispositif selon la revendication 1, **caractérisé en ce que** le récipient en forme de tube est en une pièce, où, de préférence, le réceptacle (2, 202) et la cartouche (1, 201) sont construits sous forme d'une structure synthétique thermoplastique en une pièce, où le récipient est fabriqué de préférence avec un procédé de moulage par injection.

3. Dispositif selon l'une des revendications précédentes, **caractérisé en ce que** la surface de la section transversale de l'espace intérieur de la cartouche (1, 201) est plus petite que la surface de la section transversale de l'espace intérieur du réceptacle (2, 202).

4. Dispositif selon l'une des revendications précédentes, **caractérisé en ce qu'**un système de fixation (8, 208) pour la fixation d'un dispositif d'extrusion (43) est disposé sur le récipient en forme de tube, notamment sur la face arrière du réceptacle (2, 202).

5. Dispositif selon l'une des revendications précédentes, **caractérisé en ce qu'**une butée, qui limite le mouvement du piston de transport (6, 206) en direction de la face avant, est prévue à l'intérieur du récipient, où la butée limite de préférence le mouvement de telle manière que le piston de transport (6, 206) ne peut pas être totalement pressé en dehors du réceptacle (2, 202), où, de manière particulièrement préférée, la butée est sous la forme d'un dénivelé créé par une forme variable ou des sections transversales variables de l'espace intérieur cylindrique de la cartouche (1, 201) et de l'espace intérieur cylindrique du réceptacle (2, 202)au niveau de la transition entre le réceptacle (2, 202) et la cartouche (1, 201).

6. Dispositif selon l'une des revendications précédentes, **caractérisé en ce que** la liaison (14, 214) est disposée dans le piston de sortie (7, 207) et/ou dans la paroi du récipient en forme de tube, où de préférence la liaison (14, 214) est disposée dans le piston de sortie (7, 207) et relie la face avant du piston de sortie (7, 207) avec la face arrière du piston de sortie (7, 207) orientée vers le récipient de liquide monomère (3, 203).

7. Dispositif selon l'une des revendications précédentes, **caractérisé en ce qu'**un filtre (16, 216), notamment un filtre poreux (16, 216), perméable aux gaz et au liquide de monomère (4, 204), mais imperméable pour la poudre de ciment (5, 205), est disposé à l'embouchure de la liaison (14, 214) dans l'espace intérieur de la cartouche (1, 201).

8. Dispositif selon l'une des revendications précédentes, **caractérisé en ce que** la poudre de ciment (5, 205) est adjacente à la face avant du piston de sortie (7, 207), est notamment adjacente sur toute la surface, où de préférence, la poudre de ciment (5, 205) est pressée à l'intérieur dans l'espace intérieur de la cartouche (1, 201).

9. Dispositif selon l'une des revendications précédentes, **caractérisé en ce que** la cartouche (1, 201) présente un orifice de sortie sur la face avant, qui est fermé avec une système de fermeture (36, 236), où la pâte de ciment osseux (54) peut être pressée au dehors par l'orifice de sortie lorsque l'orifice de sortie est ouvert, où le système de fermeture (36, 236) est perméable pour les gaz et imperméable pour la poudre de ciment (5, 205), où
un tube de sortie (34, 234) est disposé sur la face avant de la cartouche (1, 201), et/ou un tuyau (64) souple avec un trocart (66) est y fixé ou peut être fixé, où la pâte de ciment osseux (54) peut être pressée vers l'extérieur par le tube de sortie (34, 234) et/ou le tuyau (64) souple et le trocart (66), où, de préférence, un élément faisant soupape pouvant être fermé manuellement est disposé sur le tube de sortie (34, 234) ou le tuyau (64), avec lequel le débit de la pâte de ciment osseux (54) peut être commandé.

10. Dispositif selon l'une des revendications précédentes, **caractérisé en ce que** le piston de transport (6, 206) peut être transpercé à partir de la face arrière avec la tige (44) présentant une pointe (45) ou une arête lorsqu'un nouveau mouvement du piston de transport (6, 206) est bloqué en direction de la face avant du récipient en forme de tube, et/ou
le piston de transport (6, 206) peut être transpercé à partir de la face arrière avec la tige (44) avec une force d'au moins 1 kN lorsqu'un nouveau mouvement du piston de transport (6, 206) est bloqué en direction de la face avant du récipient en forme de tube, et/ou
le piston de transport (6, 206) a une épaisseur maximale de 4 mm, de préférence, d'au maximum 3 mm et de manière idéale d'au maximum 2 mm dans la zone de la surface d'action de la tige (44).

11. Dispositif selon l'une des revendications précédentes, **caractérisé en ce que** la section transversale de l'espace intérieur de la cartouche (1, 201) est d'au maximum 4 cm², est de préférence d'au maximum 2,5 cm², est de manière particulièrement préférée au maximum 1,2 cm², ou
le récipient de liquide de monomère (3, 203) est une ampoule en verre (3), une ampoule en plastique (3), un pochon en film synthétique (203) ou un pochon en composite aluminium-matière plastique (203).

12. Dispositif selon l'une des revendications précédentes, **caractérisé en ce que** le volume du liquide de monomère (4, 204) dans le récipient de liquide de monomère (3, 203) est au moins aussi important que le volume des espaces interstitiels remplis d'air entre les particules de poudre de ciment dans la cartouche (1, 201), de préférence, est au moins aussi important que le volume des conduites de liquide entre l'espace intérieur de la cartouche (1, 201) et l'espace intérieur du réceptacle (2, 202) plus le volume des espaces interstitiels remplis d'air entre les particules de poudre de ciment dans la cartouche (1, 201).

13. Dispositif selon l'une des revendications précédentes, **caractérisé en ce qu'**au moins
un orifice d'aération (20), qui relie l'espace intérieur du réceptacle (2, 202) dans lequel est disposé le récipient de liquide de monomère (3, 203) avec l'environnement, est disposé dans la paroi du réceptacle (2, 202), où, de préférence, l'au moins un orifice d'aération (20) est disposé de manière si proche dans la zone du piston de transport (6, 206) qu'il est fermé par un mouvement du piston de transport (6, 206) en direction de la face avant du réceptacle (2, 202) avant que le récipient de liquide de monomère (3, 203) ne soit ouvert par le mouvement du piston de transport (6, 206).

14. Dispositif selon l'une des revendications précédentes, **caractérisé en ce qu'**un tamis ou un disque poreux (18, 218), perméable pour les gaz et les liquides est prévu sur la face avant du réceptacle (2, 202) dans la liaison (14, 214) vers la cartouche (1, 201), ou
qu'une vanne à trois voies (56, 102) pouvant être actionnée de l'extérieur est disposée dans le sens de l'écoulement de la pâte de ciment osseux (54) dans la conduite derrière la cartouche (1, 201), un récipient de récupération (60, 109) pour la récupération de pâte de ciment osseux (54) est disposé au niveau de la vanne à trois voies (56, 102), où la conduite débouche dans un orifice d'application, qui est disposé à l'extrémité de la conduite opposée par rapport à la cartouche (1, 201), où la vanne à trois voies (56, 102) est construite de telle manière qu'elle procure en premier lieu une liaison de fluide entre l'orifice d'application et la cartouche (1, 201) et ferme un canal d'évacuation (88, 120) vers le récipient de récupération (60, 109), et
en second lieu, procure une liaison de fluide entre l'orifice d'application et le récipient de récupération (69, 109) et ferme un passage vers la cartouche (1, 201).

15. Dispositif selon l'une des revendications précédentes, **caractérisé en ce que** le récipient de liquide monomère (3, 203) à l'intérieur du réceptacle (2, 202) est à ouvrir, de préférence, à fracturer ou à déchirer, par un mouvement du piston de transport (6, 206) en direction de la face avant du réceptacle (2, 202), ou
le piston de transport (6, 206) qui présente un réceptacle (2, 202), ou une cavité (10), notamment une cavité (10) cylindrique au niveau de la face avant, destiné(e) à la tige (44) du dispositif d'extrusion, dans lequel (laquelle) est disposée de préférence une manchette en caoutchouc (12, 212) servant d'élément d'étanchéité ou où un disque synthétique ou métallique (9, 209) déformable de manière plastique, pouvant être transpercé par la tige (44) est inséré en tant qu'élément d'étanchéité.

16. Procédé de fabrication d'une pâte de ciment osseux (54), notamment d'une pâte de ciment osseux de poly méthyl méthacrylate (54) de forme pâteuse, où la pâte de ciment osseux (54) est fabriquée à partir d'une poudre de ciment (5, 205) et d'un liquide monomère (4, 204) à l'aide d'un dispositif, où le dispositif présente un récipient en forme de tube qui forme un réceptacle (2, 202) avec un espace intérieur cylindrique au niveau de sa face arrière, dans lequel est disposé un récipient de liquide monomère (3, 203) avec le liquide monomère (4, 204) à l'intérieur, et le récipient sur sa face avant forme une cartouche (1, 201) avec un espace intérieur cylindrique dans lequel est contenue la poudre de ciment (5, 205), **caractérisé par** les étapes suivantes se déroulant l'une après l'autre :
a) la mise en place du dispositif dans un dispositif d'extrusion (43), le dispositif d'extrusion (43) présentant une tige (44) pouvant être avancée axialement,
b) un piston de transport (6, 206), qui est logé mobile à l'intérieur du réceptacle (2, 202) au niveau de sa face arrière, est entraîné avec la tige (44) en direction de la cartouche (1, 201), où le récipient de liquide monomère (3, 203) est ouvert par le déplacement du piston de transport (6, 206) et le liquide monomère (4, 204) est pressé hors du réceptacle (2, 202) dans la cartouche (1, 201), où la poudre de ciment (5, 205) se mélange avec le liquide monomère (4, 204) dans l'espace intérieur de la cartouche (1, 201),
c) le mouvement du piston de transport (6, 206) est bloqué en direction de la face avant du récipient,
d) la tige (44) transperce le piston de transport (6, 206) et rencontre après avoir transpercé le piston de transport (6, 206) un piston de sortie (7, 207) qui est logé mobile à l'intérieur de la cartouche (1, 201),
e) le piston de transport (7, 207) est avancé avec la tige (44) en direction de la face avant de la cartouche (1, 201) tandis que la tige (44) traverse le piston de transport (6, 206) bloqué et transpercé, où le mélange à base de la poudre de ciment (5, 205) et du liquide monomère (4, 204) est expulsé de la cartouche (1, 201) sous forme de pâte de ciment osseux (54) par le mouvement du piston de sortie (7, 207).

17. Procédé selon la revendication 16, **caractérisé en ce que**
le procédé est exécuté avec un dispositif selon l'une des revendications 1 à 15.

18. Procédé selon la revendication 16 ou la revendication 17, **caractérisé en ce que**
le procédé est exécuté avec un dispositif d'extrusion (43) selon l'une des revendications 13 à 15.

19. Procédé selon l'une des revendications 16 à 18, **caractérisé en ce que** la tige (44) présente une pointe dure (45) ou une arête pour transpercer le piston de transport (6, 206) du dispositif bloqué au niveau de la face avant, où, dans l'étape a), un capuchon (46) amovible avec une face avant plane est disposé sur la pointe (45) ou l'arête, et le capuchon (46) est enlevé de la pointe (45) ou de l'arête après l'étape c), et la tige (44) est entrainée avec la pointe (45) ou l'arête dans le piston de transport (6, 206), où, de préférence, avant que le dispositif ne soit retiré du dispositif d'extrusion (43) et que le dispositif ne soit inséré de nouveau dans le dispositif d'extrusion (43) après le retrait du capuchon (46) de la pointe (45) ou de l'arête, ou
qu'avant l'étape c) ou dans l'étape c), par la pression agissant sur le mélange de la poudre de ciment (5, 205) avec le liquide monomère (4, 204), un système de fermeture (36, 236), notamment un filtre poreux (36, 236) est déplacé ou pressé vers l'avant dans un orifice de sortie au niveau de la face avant de la cartouche (1, 201), où, suite à cela, de préférence, le système de fermeture (36, 236) est retiré de l'orifice de sortie et un tube de sortie ou un tuyau (64) avec un trocart (66) est fixé sur la face avant de la cartouche (1, 201), et/ou le capuchon (46) est enlevé de la pointe (45) ou de l'arête de la tige (44).

20. Procédé selon l'une des revendications 16 à 19, **caractérisé en ce que**
le récipient de liquide monomère (3, 203) cassé, ou fendu, ou éclaté dans l'étape b) est rassemblé et simultanément du gaz provenant du réceptacle (2, 202) est pressé à travers une liaison (14, 214) dans la cartouche (1, 201) et est pressé vers l'extérieur par la poudre de ciment (5, 205) dans la cartouche (1, 201) et où, dans l'étape d), la tige (44) entrainée par le piston de transport (6, 206) repousse les morceaux cassés (52) du récipient de liquide monomère (3, 203), et/ou
dans l'étape d), immédiatement avant l'application de la pâte de ciment osseux (54) sur la face avant de la cartouche (1, 201), un trocart (66) à un orifice de sortie est relié avec un tuyau (64) avec la cartouche (1, 201), et la pâte de ciment osseux (54) est ensuite pressée à travers le tuyau (64) et le trocart (66) et éventuellement un tube de sortie (34, 234) suite au mouvement vers l'avant du piston de sortie (7, 207) par le nouvel actionnement du dispositif d'extrusion (43).
